# EUROPÄISCHE PATENTSCHRIFT

(11) **EP 2 732 479 B1**
(45) Veröffentlichungstag und Bekanntmachung des Hinweises auf die Patenterteilung: **11.09.2019**
(21) Anmeldenummer: 12731309.6
(22) Anmeldetag: 29.06.2012
(51) Int. Cl.: H01L 51/54

(54) **VERBINDUNGEN FÜR ORGANISCHE ELEKTROLUMINESZENZVORRICHTUNGEN**
COMPOUNDS FOR ORGANIC ELECTROLUMINESCENT DEVICES
COMPOSÉS POUR DES DISPOSITIFS ÉLECTROLUMINESCENTS ORGANIQUES

(30) Priorität: 11.07.2011 EP 11005644
(43) Veröffentlichungstag der Anmeldung: 21.05.2014
(73) Patentinhaber: Merck Patent GmbH, 64293 Darmstadt (DE)
(72) Erfinder: LUDEMANN, Aurélie, 60322 Frankfurt am Main (DE); JULLIART, Alice, 69300 Caluire et cuire (FR); HAYER, Anna, 55118 Mainz (DE); GERHARD, Anja, 63329 Egelsbach (DE); JOOSTEN, Dominik, 60487 Frankfurt am Main (DE); HEIL, Holger, 60389 Frankfurt am Main (DE); ECKES, Fabrice, 64293 Darmstadt (DE)
(86) Internationale Anmeldenummer: PCT/EP2012/002752
(87) Internationale Veröffentlichungsnummer: WO 2013/007348

(56) Entgegenhaltungen:
- EP-A1- 2 194 582
- WO-A1-2008/029729
- WO-A1-2011/093309
- JP-A- 2010 040 967
- US-A1- 2008 191 617
- US-A1- 2010 207 105

## Beschreibung

Die vorliegende Erfindung betrifft vernetzbare Verbindungen, die aus diesen Verbindungen erhaltenen vernetzten Verbindungen sowie Verfahren zu deren Herstellung. Die Erfindung ist ferner auf die Verwendung dieser Verbindungen in elektronischen Vorrichtungen, insbesondere in organischen Elektrolumineszenzvorrichtungen, sowie auf die entsprechenden elektronischen Vorrichtungen selbst gerichtet.

Elektronische Vorrichtungen, welche organische und/oder metallorganische Halbleiter enthalten, werden immer häufiger in kommerziellen Produkten verwendet. Als Beispiele seien hier Ladungstransportmaterialien auf organischer Basis (z. B. Lochtransportmaterialien auf Triarylaminbasis) in Kopiergeräten, organischen Leuchtdioden (OLED) oder organische Photorezeptoren in Kopierern genannt. Organische Solarzellen (O-SC), organische Feldeffekt-Transistoren (O-FET), organische Dünnfilm-Transistoren (O-TFT), organische Schaltelemente (O-IC), organische optische Verstärker und organische Laserdioden (O-Laser) sind in einem fortgeschrittenen Entwicklungsstand und können in der Zukunft große Bedeutung erlangen.

Viele dieser elektronischen Vorrichtungen weisen unabhängig vom jeweiligen Verwendungszweck den folgenden allgemeinen Schichtaufbau auf, der für die jeweilige Anwendung angepasst werden kann:
(1) Substrat,
(2) Elektrode, häufig metallisch oder anorganisch, aber auch aus organischen bzw. polymeren leitfähigen Materialien,
(3) optional Ladungsinjektionsschicht/en bzw. Zwischenschicht/en, beispielsweise zum Ausgleich von Unebenheiten der Elektrode, häufig aus einem leitfähigen, dotierten Polymer,
(4) organische Halbleiter, z. B. als emittierende Schicht,
(5) evt. weitere Ladungstransport-, Ladungsinjektions- bzw. Ladungsblockierschichten,
(6) Gegenelektrode, Materialien wie unter (2) genannt,
(7) Verkapselung.

Dabei muss für organische Elektrolumineszenzvorrichtungen mindestens eine der Elektroden transparent oder teiltransparent sein.

Die obige Anordnung stellt den allgemeinen Aufbau einer organischen elektronischen Vorrichtung dar, wobei verschiedene Schichten zusammengefasst werden können, so dass im einfachsten Fall eine Anordnung aus zwei Elektroden resultiert, zwischen denen sich eine organische Schicht befindet. Die organische Schicht erfüllt in diesem Fall alle Funktionen, einschließlich der Emission von Licht im Fall von OLEDs.

Ein Problem, das sich in einem derartigen "Dreischichtsystem" mit nur einer organischen Schicht ergibt, ist jedoch die fehlende Steuerung der Ladungstrennung und die fehlende Möglichkeit, die einzelnen Bestandteile in unterschiedlichen Schichten bezüglich ihrer Eigenschaften zu optimieren, wie es beispielsweise bei OLEDs auf Basis aufgedampfter niedermolekularer Verbindungen durch einen mehrschichtigen Aufbau einfach gelöst ist.

Eine OLED auf Basis aufgedampfter niedermolekularer Verbindungen besteht beispielsweise aus einer oder mehreren organischen Lochinjektionsschichten, Lochtransportschichten, Exzitonenblockierschichten, Emissionsschichten, Lochblockierschichten, Elektronentransportschichten und/oder Elektroneninjektionsschichten sowie einer Anode und einer Kathode. Der Vorteil einer solchen mehrlagigen Struktur besteht darin, dass die verschiedenen Funktionen der Ladungsinjektion, des Ladungstransports und der Emission in die verschiedenen Schichten aufgeteilt und somit die Eigenschaften der jeweiligen Schichten separat modifiziert und optimiert werden können.

Das Aufbringen der Schichten in einer solchen OLED erfolgt gewöhnlich durch Aufdampfen in einer Vakuumkammer. Dieses Verfahren ist jedoch aufwändig und somit teuer und insbesondere für höhermolekulare Verbindungen, wie beispielsweise Polymere, ungeeignet. Polymere OLED-Materialien werden deshalb üblicherweise durch Beschichtung aus Lösung aufgetragen. Auch für niedermolekulare organische Verbindungen wäre eine Verarbeitung aus Lösung wünschenswert wegen des hohen technischen Aufwands bei der Vakuumverarbeitung. Die Herstellung einer mehrschichtigen, organischen Struktur durch Beschichtung aus Lösung erfordert, dass das Lösungsmittel der aufzubringenden Schicht die jeweils vorhergehende Schicht nicht wieder anlöst, quellt oder gar zerstört. Die Wahl des Lösungsmittels erweist sich jedoch als schwierig, da die eingesetzten, organischen Verbindungen gewöhnlich ähnliche chemische Strukturen und Eigenschaften, insbesondere ähnliche Lösungseigenschaften, besitzen.

Entsprechend sind lösungsprozessierte OLEDs auf Basis von Polymeren oder löslichen niedermolekularen Verbindungen gemäß dem Stand der Technik gewöhnlich nur aus einer einschichtigen oder höchstens zweischichtigen, organischen Struktur aufgebaut, wobei beispielsweise eine der Schichten für die Lochinjektion und den Lochtransport und die zweite Schicht beispielsweise für die Injektion und den Transport von Elektronen sowie für die Emission verwendet wird.

Dabei hat sich gezeigt, dass es sinnvoll sein kann, zwischen der Anode bzw. einer Lochinjektionsschicht aus einem leitfähigen dotierten Polymer, z. B. PEDOT/PSS, eine löchertransportierende Zwischenschicht (Interlayer) einzufügen, die auch als Exzitonen- bzw. Elektronenblockierschicht wirken kann. Dadurch können die Eigenschaften der OLED wesentlich verbessert werden. Auf diese Zwischenschicht können dann die weiteren Schichten aufgebracht werden. Wenn die weiteren Schichten aus Lösung aufgebracht werden sollen, ist es, wie oben erwähnt, erforderlich, dass sich die löchertransportierende Zwischenschicht nicht löst.

Der Aufbau einer solchen Mehrschichtstruktur ist beispielsweise möglich, indem eine Schicht nach dem Aufbringen aus Lösung und vor dem Aufbringen der nächsten Schicht vernetzt und dadurch unlöslich gemacht wird (z. B. EP 0637899, US 6,107,452). Allerdings hat sich gezeigt, dass nicht alle chemischen Strukturen gleichermaßen hierfür geeignet sind.

Als Vernetzungsaktivierung wird generell die Temperaturbehandlung gegenüber der UV-Bestrahlung bevorzugt, da bei organischen Halbleitern, die aus Lösung aufgebracht werden, ohnehin im Allgemeinen ein thermischer Trocknungsprozess durchgeführt wird, um das Lösungsmittel zu entfernen. So kann der Vernetzungsprozess leicht in den Herstellungsprozess integriert und Schäden des halbleitenden Materials durch UV-Strahlung ausgeschlossen werden. Es ist ebenfalls bevorzugt, die Verwendung eines Initiators zu vermeiden. Für die thermische Vernetzung wird die Temperatur derart gewählt, dass die Vernetzungsreaktion stattfinden kann. Dabei ist es wünschenswert, einen Temperaturbereich zu wählen, bei dem das vernetzbare Material sowie die anderen im Schichtaufbau bereits vorhandenen Materialien sich nicht zersetzen.

Insbesondere im Fall von Blau liegen die aus Lösung prozessierten OLEDs in der Performance noch hinter denen von aufgedampften niedermolekularen Verbindungen zurück. Ein Hauptgrund hierfür wird in den Eigenschaften der Interlayer gesehen, die zwischen die Anode bzw. die Schicht aus einem leitfähigen dotierten Polymer und die emittierende Schicht eingebracht ist. Durch die Kupplung vieler aromatischer Systeme in einem Polymer lassen sich die Energielücke und die HOMO- und LUMO-Niveaus nicht so präzise wie im Fall von definierten niedermolekularen Verbindungen einstellen. Polymerdefekte, unvollständiges Endcapping, die schwierigere Aufreinigung und die Polydispersität von Polymeren werden ebenfalls als Schwachpunkte der Polymerinterlayer besonders im Fall von blau emittierenden OLEDs gesehen. Dasselbe gilt für phosphoreszierende OLEDs, insbesondere für grün phosphoreszierende OLEDs.

Es besteht ein Bedarf an Verbindungen, die Lochtransporteigenschaften aufweisen und sich somit für die Verwendung in einer Lochtransport- oder Lochinjektionsschicht eignen, gerade auch in einer Interlayer, und die zur Vernetzung geeignete Gruppen aufweisen. Vorteilhaft ist es dabei, wenn sich diese vernetzbaren Gruppen leicht, d.h. mit geringem Energieaufwand, vernetzen lassen und auch im vernetzten Zustand keine negativen Auswirkungen auf die Funktion der elektronischen Vorrichtung haben. Die Verbindungen sollten weiterhin zu vorteilhaften Eigenschaften bezüglich Effizienz, Lebensdauer und Spannung der OLED führen bzw. diese zumindest nicht verschlechtern gegenüber den entsprechenden nicht vernetzten Verbindungen bzw. gegenüber vernetzten Polymeren.

Die Aufgabe der vorliegenden Erfindung bestand somit in der Bereitstellung solcher Verbindungen.

JP 2010 040967 A offenbart Arylaminderivate, welche mit mindestens zwei Cyanovinyl vernetzbaren Gruppen substituiert sind.

Überraschend wurde gefunden, dass bestimmte unten näher beschriebene Arylaminderivate, welche mit mindestens zwei vernetzbaren Gruppen (die keine reaktiven Gruppen, wie z.B. Cyanogruppen aufweisen) substituiert sind, diese Aufgabe lösen. Mit diesen vernetzbaren Verbindungen lassen sich besonders effiziente und langlebige OLEDs aufbauen, insbesondere auch solche, welche auf Triplettemission oder auf blauer Singulettemission basieren. Die vernetzbaren Verbindungen lassen sich auf dem Substrat bzw. auf einer Schicht aus einem leitfähigen dotierten Polymer thermisch oder optisch mit oder ohne Initiator vernetzen und erlauben auf diese Weise das kontrollierte Aufbringen einer weiteren Schicht aus Lösung. Dieser Vorgang kann auch mehrmals wiederholt werden, wobei hierfür entweder die gleiche oder unterschiedliche vemetzbare Verbindungen verwendet werden können.

Gegenstand der vorliegenden Erfindung ist daher eine Verbindung der folgenden Formel (1), 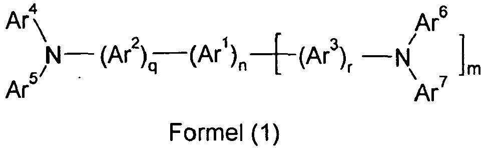 wobei für die verwendeten Symbole und Indices gilt:
- Ar¹: ist gleich oder verschieden bei jedem Auftreten eine Gruppe der folgenden Formel (2), 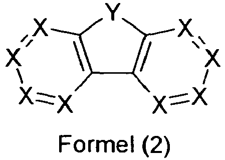 wobei die Struktur der Formel (2) an beliebigen Positionen mit Ar² und Ar³ bzw. mit N bzw. mit weiteren Gruppen Ar¹ für n > 1 verknüpft sein kann;
- Y: ist N, wenn die Gruppe der Formel (2) über dieses N mit Ar² bzw. Ar³ bzw. N bzw. Ar¹ für n > 1 verknüpft ist, oder ist gleich oder verschieden bei jedem Auftreten NR, O, S, CR=CR, CR₂-CR₂ oder eine Gruppe der folgenden Formel (3), 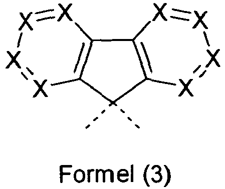 wobei die gestrichelten Bindungen die Verknüpfung der Gruppe kennzeichnen;
oder Y kann weiterhin auch für CR₂ stehen, wenn zwei benachbarten Gruppen X zusammen für eine Gruppe der Formel (4), (5) oder (6) stehen;
- X: ist C, wenn die Gruppe der Formel (2) über dieses X mit Ar² bzw. Ar³ bzw. N bzw. Ar¹ für n > 1 verknüpft ist, oder ist gleich oder verschieden bei jedem Auftreten CR oder N; oder zwei benachbarte Gruppen X stehen zusammen für eine Gruppe der folgenden Formel (4), (5) oder (6), 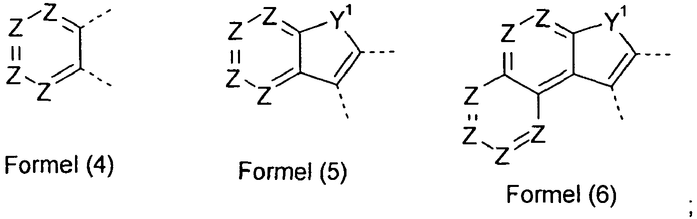 wobei die gestrichelten Bindungen die Verknüpfung der Gruppe kennzeichnen;
- Z: ist C, wenn die Gruppe der Formel (4) bzw. Formel (5) bzw. Formel (6) über dieses X mit Ar² bzw. Ar³ bzw. N bzw. Ar¹ für n > 1 verknüpft ist, oder ist bei jedem Auftreten gleich oder verschieden CR oder N;
- Y¹: ist bei jedem Auftreten gleich oder verschieden CR₂, NR, O oder S;
- Ar², Ar³: ist bei jedem Auftreten gleich oder verschieden eine Aryl- oder Heteroarylgruppe mit 5 bis 18 aromatischen Ringatomen, welche mit einem oder mehreren Resten R substituiert sein kann;
- Ar⁴ bis Ar⁷: ist bei jedem Auftreten gleich oder verschieden ein aromatisches oder heteroaromatisches Ringsystem mit 5 bis 24 aromatischen Ringatomen, welches durch einen oder mehrere Reste R substituiert sein kann;
- R: ist bei jedem Auftreten gleich oder verschieden H, D, F, Cl, Br, I, CN, NO₂, Si(R¹)₃, B(OR¹)₂, C(=O)R¹, P(=O)(R¹)₂, S(=O)R¹, S(=O)₂R¹, OSO₂R¹, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R¹ substituiert sein kann, wobei eine oder mehrere nicht benachbarte CH₂-Gruppen durch R¹C=CR¹, C≡C, Si(R¹)₂, C=O, C=S, C=NR¹, P(=O)(R¹), SO, SO₂, NR¹, O, S oder CONR¹ ersetzt sein können und wobei ein oder mehrere H-Atome durch D, F, Cl, Br, I oder CN ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 60 aromatischen Ringatomen, die durch einen oder mehrere Reste R¹ substituiert sein kann, oder eine Aralkyl- oder Heteroaralkylgruppe mit 5 bis 60 aromatischen Ringatomen, die durch einen oder mehrere Reste R¹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R¹ substituiert sein kann; dabei können zwei oder mehrere Reste R auch miteinander ein mono- oder polycyclisches, aliphatisches, aromatisches und/oder benzoannelliertes Ringsystem bilden;
- R¹: ist bei jedem Auftreten gleich oder verschieden H, D, F oder ein aliphatischer, aromatischer und/oder heteroaromatischer Kohlenwasserstoffrest mit 1 bis 20 C-Atomen, in dem auch ein oder mehrere H-Atome durch F ersetzt sein können; dabei können zwei oder mehrere Substituenten R¹ auch miteinander ein mono- oder polycyclisches, aliphatisches oder aromatisches Ringsystem bilden;
- n: ist 2, 3 oder 4;
- m: ist 1;
- q, r: ist gleich oder verschieden bei jedem Auftreten 0, 1, 2 oder 3;
die dadurch gekennzeichnet ist, dass mindestens zwei der Gruppen Ar⁴ bis Ar⁷ jeweils durch eine Gruppe der folgenden Formel (7) substituiert sind:

----L-(Ar⁸)ₚ-Q Formel (7)

worin gilt:
- L: ist gleich oder verschieden bei jedem Auftreten eine Abstandsgruppe oder eine direkte Bindung;
- Ar⁸: ist gleich oder verschieden bei jedem Auftreten ein aromatisches oder heteroaromatisches Ringsystem mit 5 bis 24 aromatischen Ringatomen, welches durch einen oder mehrere Reste R substituiert sein kann;
- Q: ist gleich oder verschieden bei jedem Auftreten eine vernetzbare Gruppe, ausgewählt aus endständigen oder cyclischen Alkenylgruppen, endständigen Alkinylgruppen, Arylvinylgruppen, Acrylsäurederivaten, Alkenyloxy- bzw. Perfluoralkenyloxyderivate, Gruppen, die eine Ringöffnungspolymerisation eingehen, insbesondere Oxetan- und Oxiranderivaten, oder Silanen;
- p: ist gleich oder verschieden bei jedem Auftreten 0 oder 1.

"Vernetzbare Gruppe" im Sinne der vorliegenden Erfindung bedeutet eine funktionelle Gruppe, die in der Lage ist, eine Reaktion, vorzugsweise eine Polymerisationsreaktion, einzugehen und so eine unlösliche Verbindung zu bilden. Bei der vernetzbaren Gruppe handelt es sich somit um eine polymerisierbare Gruppe. Dabei erhält man als Ergebnis der Reaktion der vernetzbaren Gruppe eine entsprechende vernetzte Verbindung. Die chemische Reaktion kann auch in der Schicht durchgeführt werden, wobei eine unlösliche Schicht entsteht. Die Vernetzung kann gewöhnlich durch Wärme oder durch UV-, Mikrowellen-, Röntgen- oder Elektronenstrahlung unterstützt werden, gegebenenfalls in Gegenwart eines Initiators. "Unlöslich" im Sinne der vorliegenden Erfindung bedeutet vorzugsweise, dass die Verbindung nach der Vernetzungsreaktion, also nach der Reaktion der vernetzbaren Gruppen, bei Raumtemperatur in Toluol eine Löslichkeit aufweist, die mindestens einen Faktor 3, vorzugsweise mindestens einen Faktor 10 geringer ist als die der nicht-vernetzten Verbindung gemäß Formel (1).

Eine Arylgruppe im Sinne der vorliegenden Erfindung enthält 6 bis 40 C-Atome; eine Heteroarylgruppe im Sinne der vorliegenden Erfindung enthält 2 bis 40 C-Atome und mindestens ein Heteroatom, mit der Maßgabe, dass die Summe aus C-Atomen und Heteroatomen mindestens 5 ergibt. Die Heteroatome sind vorzugsweise ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne der vorliegenden Erfindung enthält 6 bis 60 C-Atome im Ringsystem. Ein heteroaromatisches Ringsystem im Sinne der vorliegenden Erfindung enthält 1 bis 60 C-Atome und mindestens ein Heteroatom im Ringsystem, mit der Maßgabe, dass die Summe aus C-Atomen und Heteroatomen mindestens 5 ergibt. Die Heteroatome sind vorzugsweise ausgewählt aus N, O und/oder S. Unter einem aromatischen oder heteroaromatischen Ringsystem im Sinne der vorliegenden Erfindung soll ein System verstanden werden, das nicht notwendigerweise nur Aryl- oder Heteroarylgruppen enthält, sondern in dem auch mehrere Aryl- oder Heteroarylgruppen durch eine nichtaromatische Einheit (vorzugsweise weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne der vorliegenden Erfindung verstanden werden, und ebenso Systeme, in denen zwei oder mehrere Arylgruppen beispielsweise durch eine lineare oder cyclische Alkylgruppe oder durch eine Silylgruppe unterbrochen sind.

Unter einer cyclischen Alkyl-, Alkoxy- oder Thioalkoxygruppe im Sinne der vorliegenden Erfindung wird eine monocyclische, eine bicyclische oder eine polycyclische Gruppe verstanden.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, beispielsweise die Reste Methyl, Ethyl, n-Propyl, i-Propyl, n-Butyl, i-Butyl, s-Butyl, t-Butyl, 2-Methylbutyl, n-Pentyl, s-Pentyl, tert-Pentyl, 2-Pentyl, Cyclopentyl, n-Hexyl, s-Hexyl, tert-Hexyl, 2-Hexyl, 3-Hexyl, Cyclohexyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Trifluormethyl, Pentafluorethyl oder 2,2,2-Trifluorethyl verstanden.

Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden.

Unter einer Alkinylgruppe werden beispielsweise Ethinyl, Propinyl, Butinyl, Pentinyl, Hexinyl, Heptinyl oder Octinyl verstanden. Unter einer C₁- bis C₄₀-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 bis 60 aromatischen Ringatomen, welches noch jeweils mit den oben genannten Resten R substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzindenofluoren, cis- oder trans-Dibenzoindenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

In einer bevorzugten Ausführungsform der vorliegenden Erfindung ist die Gruppe Ar¹ gleich oder verschieden bei jedem Auftreten ausgewählt aus den Gruppen der folgenden Formeln (8) bis (27), 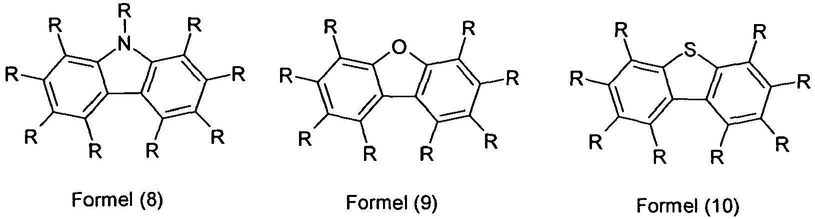 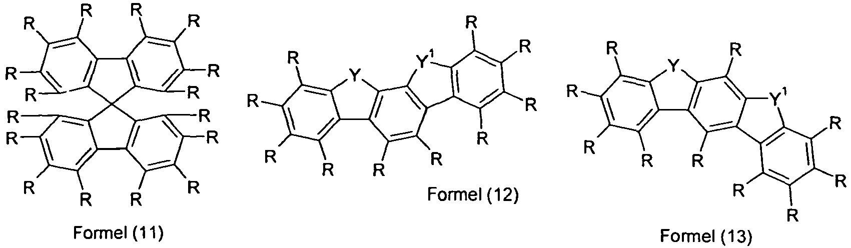 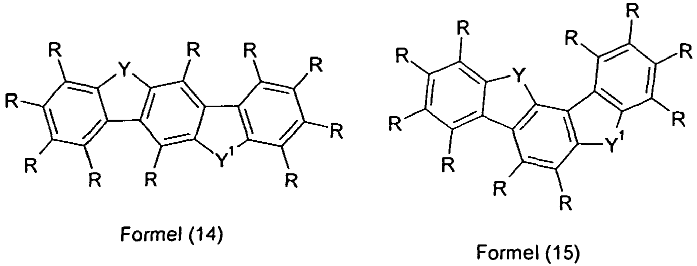 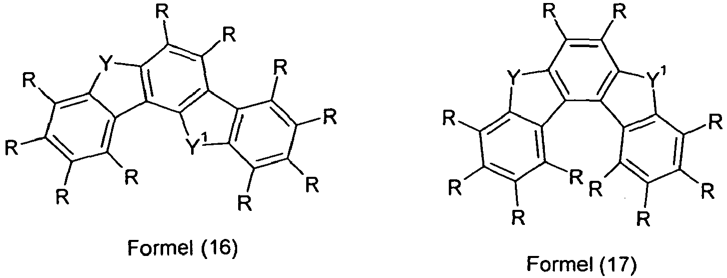 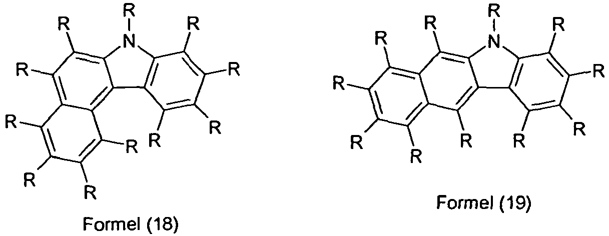 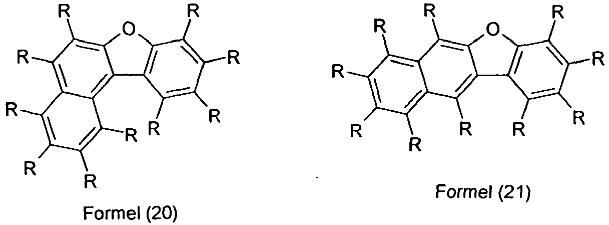 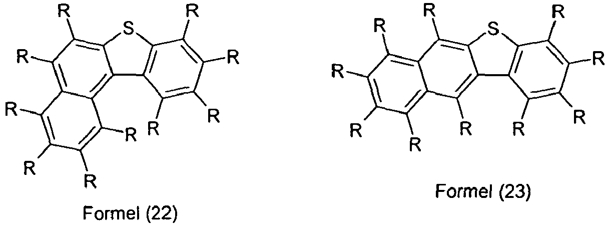 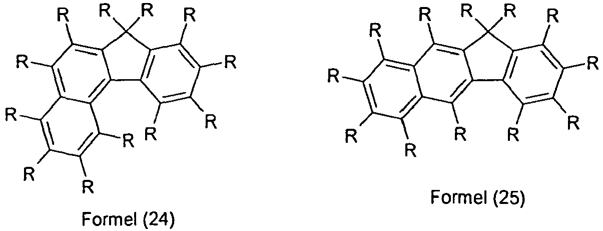 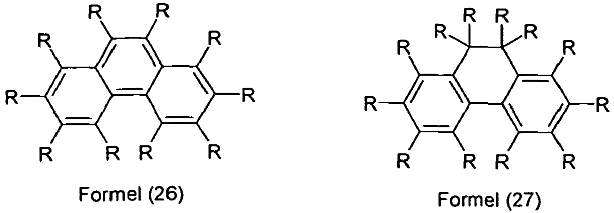 wobei Y, Y¹ und R die oben genannten Bedeutungen aufweisen und kein Rest R an den Positionen vorhanden ist, an denen die Struktur an Ar² bzw. Ar³ bzw. an den Stickstoff bzw. an eine weitere Gruppe Ar¹ für n > 1 gebunden ist. Dabei können die Gruppen jeweils über beliebige Positionen binden.

Besonders bevorzugte Gruppen Ar¹ sind gleich oder verschieden bei jedem Auftreten die Strukturen der folgenden Formeln (8a) bis (27b), 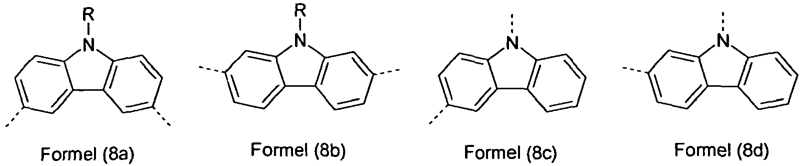 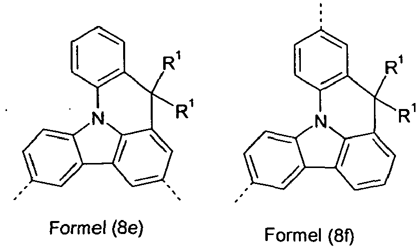 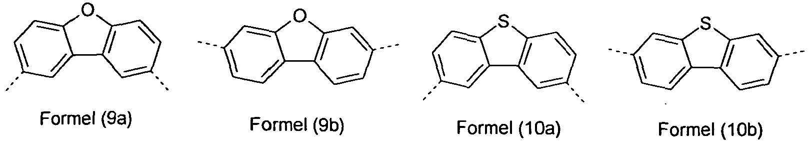 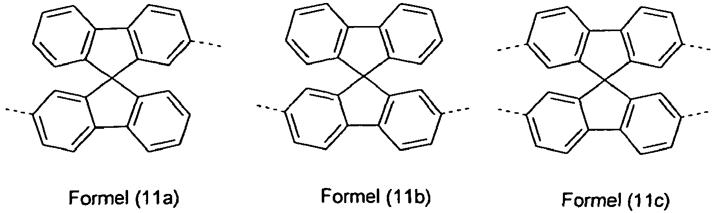 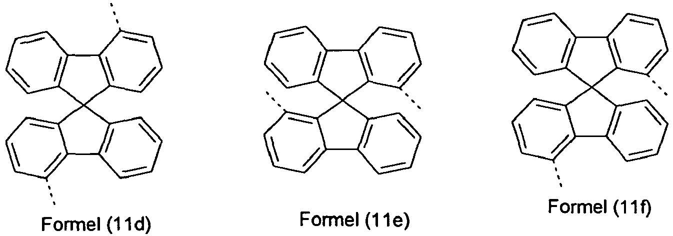 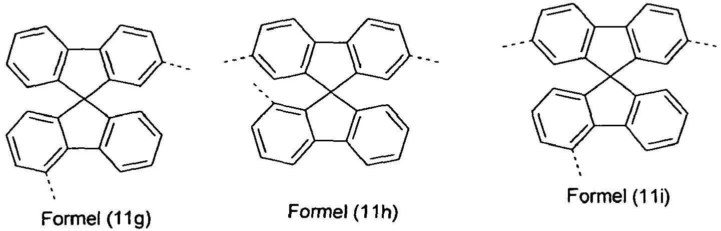 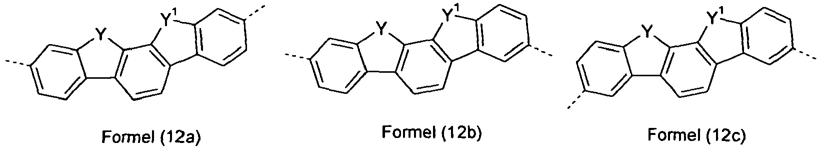 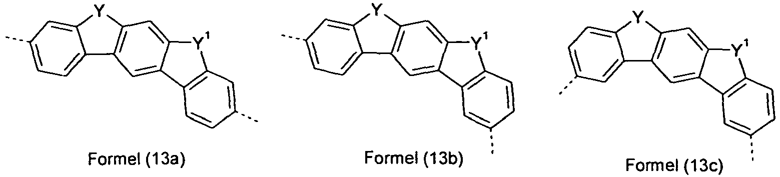 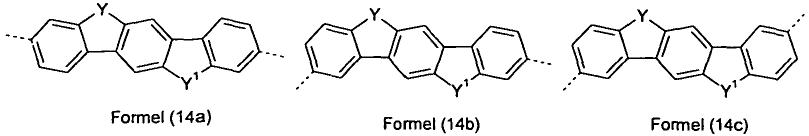 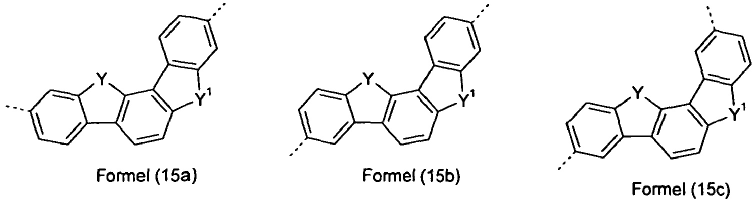 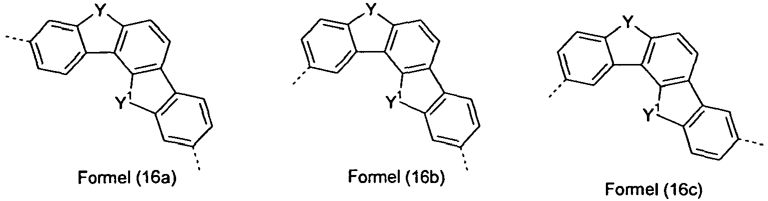 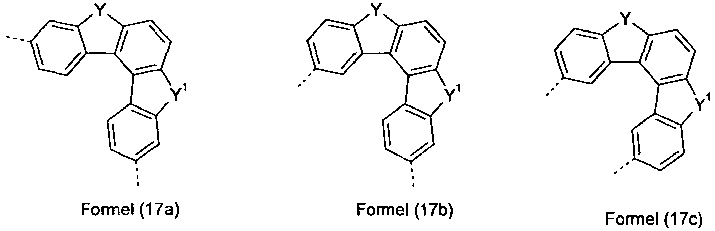 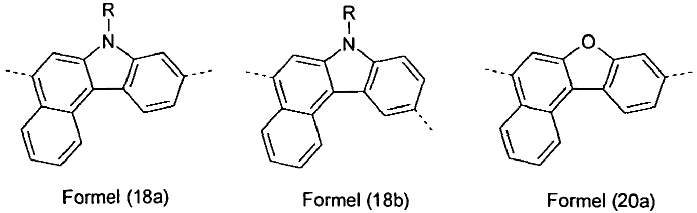 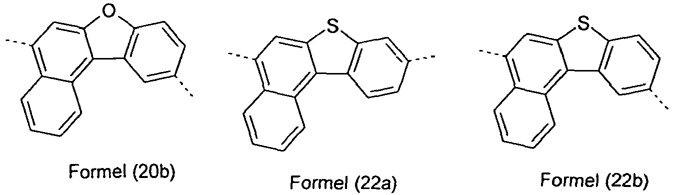 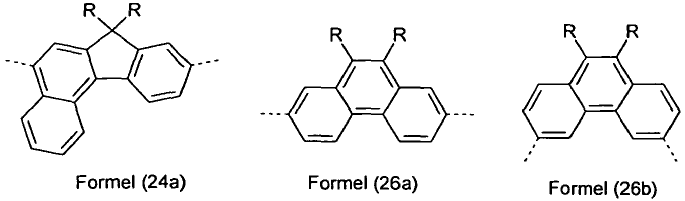 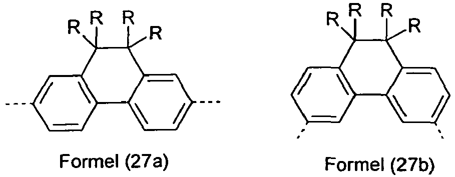 wobei Y, Y¹ und R die oben genannten Bedeutungen aufweisen und die gestrichelte Bindung die Position andeutet, an denen die Struktur an Ar² bzw. Ar³ bzw. an den Stickstoff bzw. an Ar¹ für n > 1 gebunden ist. Wenn die oben genannte Gruppe zwei gestrichelte Bindungen aufweist, steht der Index m für 1, und wenn die oben genannte Gruppe vier gestrichelte Bindungen aufweist, steht der Index m für 3.

In einer bevorzugten Ausführungsform der Erfindung ist der Index n = 2 oder 3, besonders bevorzugt 2.

Wenn n = 2 oder 3 ist, ist Ar¹ gleich oder verschieden bei jedem Auftreten, bevorzugt gleich, bevorzugt ausgewählt aus den Strukturen der oben genannten Formeln (8a) bis (8d), (9a), (9b), (10a), (10b), (11a) bis (11c), (18a), (18b), (19a), (19b), (24a) und (25a). Dabei erfolgt die Verknüpfung der Strukturen miteinander an einer der Positionen, die durch eine gestrichelte Bindung gekennzeichnet sind.

Besonders bevorzugte Einheiten Ar¹ sind solche, durch die die Konjugation zwischen den Stickstoffatomen in der Verbindung der Formel (1) unterbrochen oder zumindest teilweise unterbrochen wird. Eine solche Konjugationsunterbrechung erhält man beispielsweise durch ein sp³-hybridisiertes Kohlenstoffatom, wie in den Einheiten der Formel (11a). Eine teilweise Unterbrechung der Konjugation erhält man auch, wenn die Verknüpfung der Einheit gemäß Formel (2) in den para-Positionen zur Gruppe Y erfolgt, wie in den Einheiten der Formel (8a), (8e), (9a) und (10a), oder wenn die Verknüpfung der Einheit gemäß Formel (2) über die Gruppe Y erfolgt, wie in den Einheiten der Formel (8c), (8d) oder (8f).

Besonders bevorzugte Einheiten Ar¹ sind daher die Einheiten der Formeln (8a), (8c), (8d), (8e), (8f), (9a), (10a), (11a) und (11c). Ganz besonders bevorzugt sind die Einheiten der Formeln (8a), (9a), (10a), (11a) und (11c), insbesondere die Einheiten der Formel (8a), (11a) und (11c).

In einer weiteren bevorzugten Ausführungsform der vorliegenden Erfindung sind die Indices q und r gleich oder verschieden bei jedem Auftreten 0 oder 1, d. h. die Gruppe Ar¹ ist entweder direkt an den Stickstoff gebunden oder ist über eine einzelne Aryl- bzw. Heteroarylgruppe Ar² bzw. Ar³ an den Stickstoff gebunden.

Ar² bzw. Ar³ ist für q bzw. r ungleich 0 gleich oder verschieden bei jedem Auftreten, bevorzugt gleich, bevorzugt ausgewählt aus 1,2-Phenylen, 1,3-Phenylen oder 1,4-Phenylen, welches jeweils unsubstituiert oder durch einen oder mehrere Reste R substituiert sein kann. Besonders bevorzugt ist Ar² bzw. Ar³ für q bzw. r ungleich 0 1,3-Phenylen oder 1,4-Phenylen, welches unsubstituiert oder durch einen oder mehrere Reste R substituiert sein kann. Wenn Ar² bzw. Ar³ durch einen oder mehrere Reste R substituiert ist, dann sind diese Reste R vorzugsweise Alkylgruppen mit 1 bis 10 C-Atomen, besonders bevorzugt mit 1 bis 4 C-Atomen.

In einer weiteren bevorzugten Ausführungsform der vorliegenden Erfindung ist Ar⁴ bis Ar⁷ gleich oder verschieden bei jedem Auftreten ein aromatisches oder heteroaromatisches Ringsystem mit 6 bis 24 aromatischen Ringatomen, welches nicht mehr als zwei direkt aneinander kondensierte aromatische oder heteroaromatische Sechsringe enthält. Dabei können Ar⁴ bis Ar⁷ auch durch einen oder mehrere Reste R substituiert sein, wie oben beschrieben. Besonders bevorzugt enthält Ar⁴ bis Ar⁷ überhaupt keine direkt aneinander kondensierten aromatischen oder heteroaromatischen Sechsringe. Ganz besonders bevorzugt ist Ar⁴ bis Ar⁷ gleich oder verschieden bei jedem Auftreten ausgewählt aus der Gruppe bestehend aus Phenyl, ortho-, meta- oder para-Biphenyl, ortho-, meta- oder para-Terphenyl, lineares oder verzweigtes Quaterphenyl, Fluorenyl, Spirobifluorenyl oder Carbazolyl, welches jeweils durch einen oder mehrere Reste R substituiert sein kann. Ganz besonders bevorzugt ist mindestens eine der Gruppen Ar⁴ bis Ar⁷ ausgewählt aus der Gruppe bestehend aus Biphenyl oder Fluorenyl, welches jeweils auch durch einen oder mehrere Reste R substituiert sind kann. Wenn die Gruppe Ar⁴ bis Ar⁷ einen Rest R aufweist, so ist dieser Rest R vorzugsweise ausgewählt aus einer Alkylgruppe mit 1 bis 10 C-Atomen, besonders bevorzugt mit 1 bis 4 C-Atomen.

Die Verbindung gemäß Formel (1) ist, wie oben beschrieben, mit mindestens zwei Gruppen der Formel (7) substituiert. Vorzugsweise enthält die Verbindung gemäß Formel (1) zwischen 2 und 8 Gruppen der Formel (7), besonders bevorzugt 2, 3 oder 4 Gruppen der Formel (7), ganz besonders bevorzugt genau 2 Gruppen der Formel (7). Bei den Gruppen der Formel (7) handelt es sich um vernetzbare Einheiten Q, die entweder direkt oder über eine Abstandsgruppe L und/oder eine aromatische Einheit Ar⁸ verknüpft sind.

In einer Ausführungsform der vorliegenden Erfindung ist die Gruppe L eine Einfachbindung.

In einer weiteren Ausführungsform der vorliegenden Erfindung ist die Gruppe L eine so genannte Abstandsgruppe, auch Spacer genannt. Als Abstandsgruppe L können alle Gruppen eingesetzt werden, die dem Fachmann für diesen Zweck bekannt sind. Dabei erfüllt die Gruppe L einerseits die Aufgabe, die vernetzbare Gruppe Q elektronisch vom Rest des Moleküls zu entkoppeln. Weiterhin wird durch die Gruppe L Flexibilität in die erfindungsgemäße Verbindung eingeführt, was die Durchführung der Vernetzungsreaktion unterstützt.

Wenn L eine Abstandsgruppe ist, ist L vorzugsweise eine lineare oder verzweigte Alkylen-Gruppe mit 1 bis 20 C-Atomen, besonders bevorzugt mit 1 bis 12 C-Atomen, in der eine oder mehrere nicht benachbarte CH₂-Gruppen durch -O-, -S-, -NH-, -N(CH₃)-, -N-CO-, -N-CO-O-, -N-CO-N, -CO-, -O-CO-, -S-CO-, -O-COO-, -CO-S-, -CO-O-, -CH(Halogen)-, -CH(CN)-, -CH=CH- oder -C≡C- ersetzt sein können, oder eine cyclische Alkylgruppe, vorzugsweise Cyclohexan oder ein Cyclohexanderivat mit 1,4- oder 1,3-Verknüpfung. Weitere mögliche Abstandsgruppen L sind zum Beispiel -(CH₂)ₛ-, -(CH₂CH₂O)ₜ-CH₂CH₂-, -CH₂CH₂-S-CH₂CH₂- oder -CH₂CH₂-NH-CH₂CH₂-, mit s = 2 bis 12 und t = 1 bis 3, aber auch -O-.

Bevorzugt ist, dass L eine Einfachbindung oder eine Alkylen- oder Alkylenoxy-Gruppe mit 2 bis 8 C-Atomen bedeutet. Hierbei sind geradkettige Gruppen besonders bevorzugt.

Besonders bevorzugte Gruppen L sind eine Einfachbindung, Ethylen, Propylen, Butylen, Pentylen, Hexylen, Heptylen, Octylen, Nonylen, Decylen, Undecylen, Dodecylen, Octadecylen, Ethylenoxyethylen, Methylenoxybutylen, Ethylenthioethylen, Ethylen-N-methyl-iminoethylen, 1-Methylalkylen, Ethenylen, Propenylen oder Butenylen.

Ar⁸ ist für p ungleich 0 vorzugsweise ausgewählt aus 1,2-Phenylen, 1,3-Phenylen oder 1,4-Phenylen, welches jeweils unsubstituiert oder durch einen oder mehrere Reste R substituiert sein kann. Besonders bevorzugt ist Ar⁸ 1,4-Phenylen, welches unsubstituiert durch einen oder mehrere Reste R substituiert sein kann. Wenn Ar⁸ durch einen oder mehrere Reste R substituiert ist, dann sind diese Reste vorzugsweise Alkylgruppen mit 1 bis 10 C-Atomen, besonders bevorzugt mit 1 bis 4 C-Atomen.

Wie oben beschrieben, handelt es sich bei der vernetzbaren Gruppe Q um eine funktionelle Gruppe, die in der Lage ist, eine chemische Reaktion einzugehen und so eine unlösliche Verbindung zu bilden. Dabei handelt es sich bei der chemischen Reaktion um eine Polymerisationsreaktion. Es ist insbesondere die Aufgabe dieser Gruppe, durch eine Vernetzungsreaktion die erfindungsgemäßen Verbindungen, gegebenenfalls mit weiteren reaktiven Verbindungen, miteinander zu verknüpfen. Dies führt zu einer vernetzten Verbindung, bzw., wenn die Reaktion in einer Schicht durchgeführt wird, zu einer vernetzten Schicht. Unter einer vernetzten Schicht im Sinne der vorliegenden Erfindung wird eine Schicht verstanden, die erhältlich ist durch Durchführung der Vernetzungsreaktion aus einer Schicht der erfindungsgemäßen Verbindung. Es handelt sich bei der Vernetzungsreaktion um eine Polymerisationsreaktion im klassischen Sinn, also um eine Kettenreaktion, bei der ein vernetztes Polymer gebildet wird. Die Vernetzungsreaktion kann im Allgemeinen durch Wärme und/oder durch UV-, Mikrowellen-, Röntgen- oder Elektronenstrahlung und/oder durch den Einsatz von Radikalbildnern, Anionen, Kationen, Säuren und/oder Photosäuren initiiert werden. Ebenso kann die Anwesenheit von Katalysatoren sinnvoll oder notwendig sein. Bevorzugt ist die Vernetzungsreaktion eine Reaktion, für die kein Initiator und kein Katalysator zugesetzt werden muss.

Erfindungsgemäß vernetzbare Gruppen Q sind die im Folgenden aufgeführten Einheiten.

### a) Endständige oder cvclische Alkenyl- bzw. endständige Alkinylgruppen:

Es eignen sich Einheiten, die eine endständige oder cyclische Doppel- oder eine endständige Dreifachbindung enthalten, insbesondere endständige oder cyclische Alkenyl- bzw. endständige Alkinylgruppen mit 2 bis 40 C-Atomen, bevorzugt mit 2 bis 10 C-Atomen, wobei auch einzelne CH₂-Gruppen und/oder einzelne H-Atome durch die oben bei R genannten Gruppen ersetzt sein können. Dabei können in den endständigen Alkenyl- bzw. Alkinylgruppen auch einzelne CH₂-Gruppen durch die oben bei R genannten Gruppen ersetzt sein. Weiterhin eignen sich auch Gruppen, die als Vorstufen zu betrachten sind und die in situ zu einer Bildung einer Doppel- oder Dreifachbindung in der Lage sind.

Bevorzugte vernetzbare Gruppen Q umfassen Vinyl, Propenyl, Butenyl, C₄₋₂₀-Cycloalkenyl und Ethinyl. So eignen sich beispielsweise die im Folgenden aufgeführten Gruppen, wobei in diesen Gruppen jeweils durch die gestrichelte Bindung die Anknüpfung an Ar⁸ bzw. an L bzw. an eine der Gruppen Ar⁴ bis Ar⁷ angedeutet ist und diese Gruppen jeweils durch einen oder mehrere Reste R substituiert sein können, jedoch vorzugsweise unsubstituiert sind:

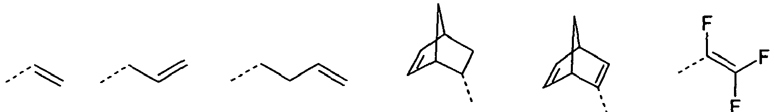

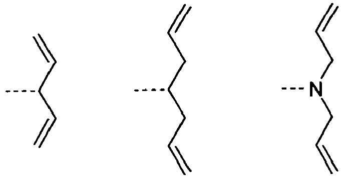

Weiterhin eignen sich Arylvinylgruppen im weitesten Sinne. Unter einer Arylvinylgruppe im Sinne der vorliegenden Anmeldung wird eine Aryl- oder Heteroarylgruppe verstanden, welche durch eine Vinylgruppe substituiert ist und welche auch einen oder mehrere weitere Reste R tragen kann. So eignen sich beispielsweise die im Folgenden aufgeführten Gruppen, wobei in diesen Gruppen jeweils durch die gestrichelte Bindung die Anknüpfung an Ar⁸ bzw. an L bzw. an eine der Gruppen Ar⁴ bis Ar⁷ angedeutet ist und diese Gruppen jeweils durch einen oder mehrere Reste R substituiert sein können, jedoch vorzugsweise unsubstituiert sind:

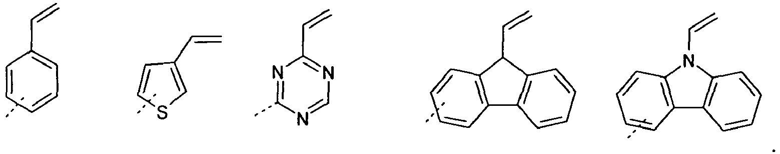

Dabei ist eine Styrylgruppe, also die als erstes aufgeführte Gruppe, besonders bevorzugt.

Weiterhin eignen sich Acrylsäurederivate im weitesten Sinne, insbesondere Acrylester, Acrylamide, Methacrylester und Methacrylamide.

Besonders bevorzugt sind C₁₋₁₀-Alkylacrylat und C₁₋₁₀-Alkylmethacrylat. So eignen sich beispielsweise die im Folgenden aufgeführten Gruppen, wobei in diesen Gruppen jeweils durch die gestrichelte Bindung die Anknüpfung an Ar⁸ bzw. an L bzw. an eine der Gruppen Ar⁴ bis Ar⁷ angedeutet ist und diese Gruppen jeweils durch einen oder mehrere Reste R substituiert sein können, jedoch vorzugsweise unsubstituiert sind:

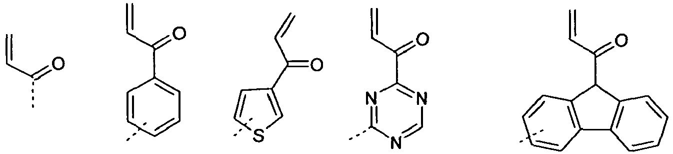

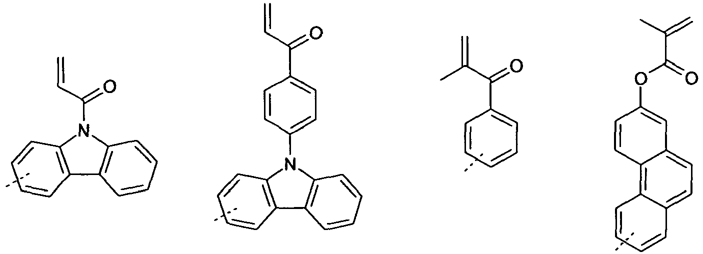

Weiterhin eignen sich Alkenyloxy- bzw. Perfluoralkenyloxyderivate, insbesondere Ethenylenoxy oder Perfluorethenylenoxy. So eignen sich beispielsweise die im Folgenden aufgeführten Gruppen, wobei in diesen Gruppen jeweils durch die gestrichelte Bindung die Anknüpfung an Ar⁸ bzw. an L bzw. an eine der Gruppen Ar⁴ bis Ar⁷ angedeutet ist und diese Gruppen jeweils durch einen oder mehrere Reste R substituiert sein können, jeodch vorzugsweise unsubstituiert sind:

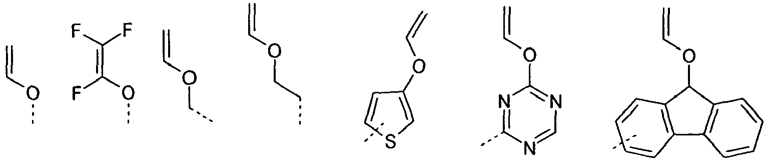

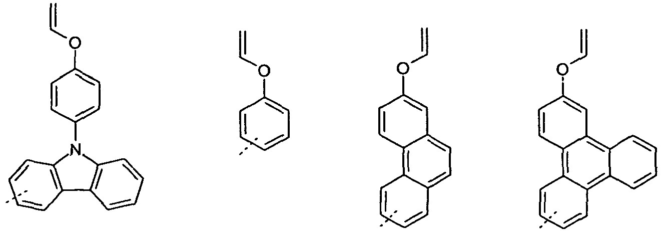

Die Polymerisationsreaktion der oben genannten Alkenyl- bzw. Alkinylgruppen kann über einen radikalischen, einen kationischen oder einen anionischen Mechanismus erfolgen.

Es kann sinnvoll sein, einen entsprechenden Initiator für die Polymerisationsreaktion zuzugeben. Geeignete Initiatoren für die radikalische Polymerisation sind beispielsweise Dibenzoylperoxid, AIBN oder TEMPO. Geeignete Initiatoren für die kationische Polymerisation sind beispielsweise AlCl₃, BF₃, Triphenylmethylperchlorat, Tropyliumhexachlorantimonat, etc.. Geeignete Initiatoren für die anionische Polymerisation sind Basen, insbesondere Butyllithium.

In einer bevorzugten Ausführungsform der vorliegenden Erfindung wird die Polymerisation jedoch ohne den Zusatz eines Initiators durchgeführt und ausschließlich thermisch initiiert. Diese Bevorzugung wird dadurch begründet, dass die Abwesenheit des Initiators Verunreinigungen der Schicht verhindert, die zu einer Verschlechterung der Deviceeigenschaften führen könnten.

### b) Ringöffnungspolymerisation auf Basis von Oxetanen und Oxiranen

Eine weitere Klasse vernetzbarer Gruppen Q sind Oxetane und Oxirane, die durch Ringöffnung kationisch polymerisieren. So eignen sich beispielsweise die im Folgenden aufgeführten Gruppen, wobei in diesen Gruppen jeweils durch die gestrichelte Bindung die Anknüpfung an Ar⁸ bzw. an L bzw. an eine der Gruppen Ar⁴ bis Ar⁷ angedeutet ist und diese Gruppen jeweils durch einen oder mehrere Reste R substituiert sein können, jedoch vorzugsweise unsubstituiert sind; R' steht für eine Methyl- oder Ethylgruppe:

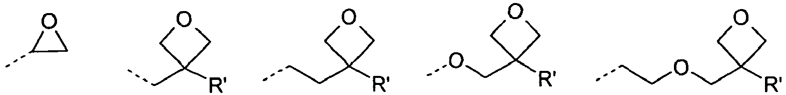

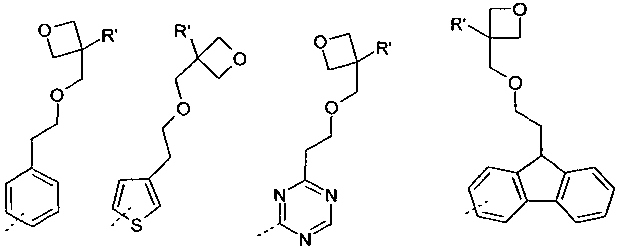

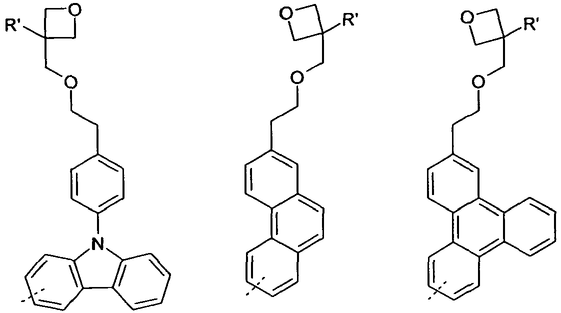

Es kann sinnvoll sein, einen entsprechenden Initiator für die Polymerisationsreaktion zuzugeben. Geeignete Initiatoren sind beispielsweise AlCl₃, BF₃, Triphenylmethylperchlorat, Tropyliumhexachlorantimonat, etc.. Ebenso können Photosäuren als Initiatoren zugegeben werden.

### c) Silane

Weiterhin ist Q ausgewählt aus Silangruppen SiR₃, wobei mindestens zwei Gruppen R, bevorzugt alle drei Gruppen R für Cl oder eine Alkoxygruppe mit 1 bis 20 C-Atomen steht. Diese Gruppe reagiert in der Anwesenheit von Wasser zu einem Oligo- oder Polysiloxan. Die oben genannten vernetzbaren Gruppen Q sind dem Fachmann auf dem Gebiet der Polymerchemie generell bekannt, ebenso wie die geeigneten Reaktionsbedingungen, die zur Reaktion dieser Gruppen verwendet werden.

In einer bevorzugten Ausführungsform sind die vernetzbaren Gruppen Q gewählt aus endständigen oder cyclischen Alkenyl- bzw. endständigen Alkinylgruppen, wie sie oben unter a) aufgeführt sind, insbesondere aus endständigen Alkenylgruppen und Arylvinylgruppen. Diese weisen den Vorteil auf, dass sie unter vergleichsweise milden Bedingungen thermisch reagieren und sich so vernetzen lassen. Eine besonders bevorzugte Alkenylgruppe ist die Vinylgruppe. Besonders bevorzugte Arylvinylgruppen sind Styrylgruppen, wobei die Vinylgruppe an der Phenylgruppe in ortho-, meta- oder para-Position gebunden sein kann, bevorzugt aber in para-Position gebunden ist.

Die oben genannten Ausführungsformen können beliebig miteinander kombiniert werden. Besonders bevorzugt treten die oben als bevorzugt genannten Ausführungsformen gleichzeitig auf.

Geeignete und bevorzugte erfindungsgemäße Verbindungen sind in der folgenden Tabelle ausgebildet. Die Verbindungen 4, 6, 7, 14, 19, 20, 23, 25, 28-30, 32-50, 53-60 sind nicht Verbindungen gemäß Formel (1) von Anspruch, weil n=1; m=2, 3; R ist N(R¹)₂ und/oder es gibt eine Verknüpfung zwischen Ar⁴ und Ar⁵ oder zwischen Ar⁶ und Ar⁷.

| | |
|---|---|
| 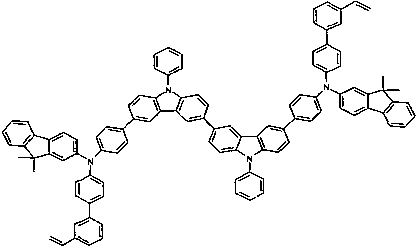 | 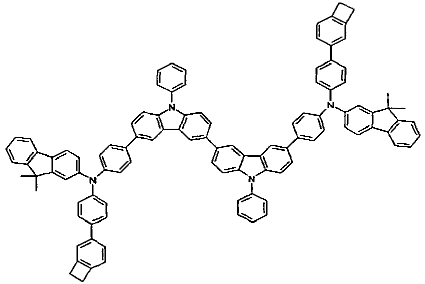 |
| (1) | (2) |
| 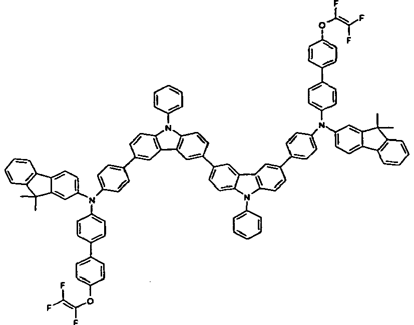 | 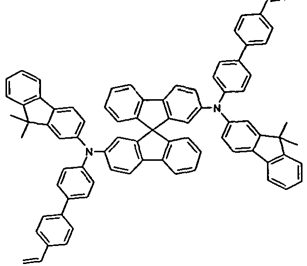 |
| (3) | (4) |
| 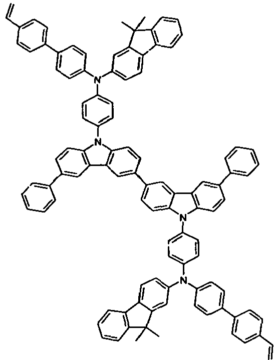 | 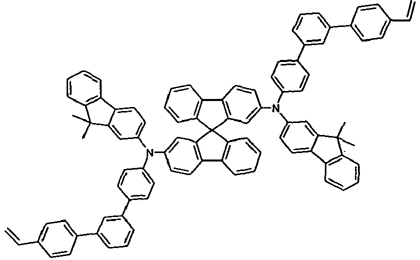 |
| (5) | (6) |
| 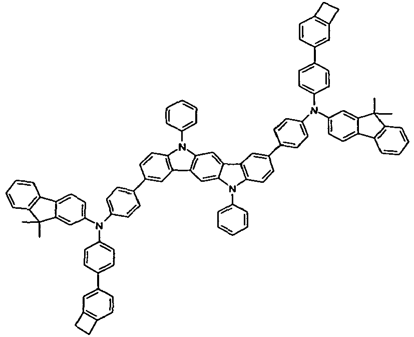 | 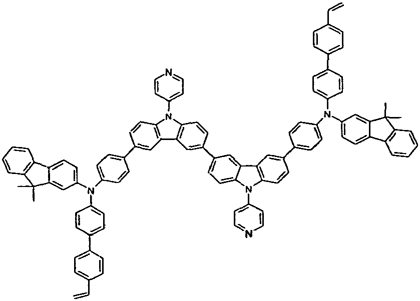 |
| (7) | (8) |
| 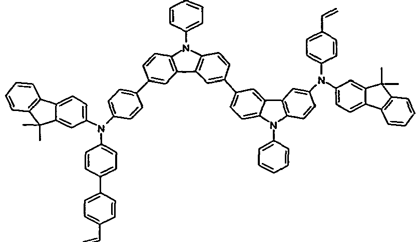 | 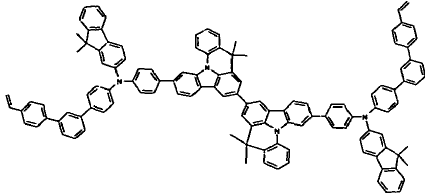 |
| (9) | (10) |
| 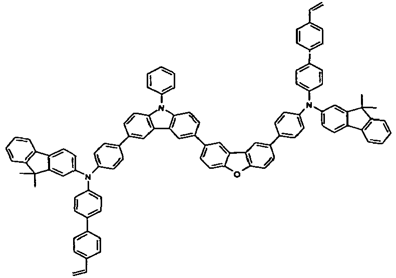 | 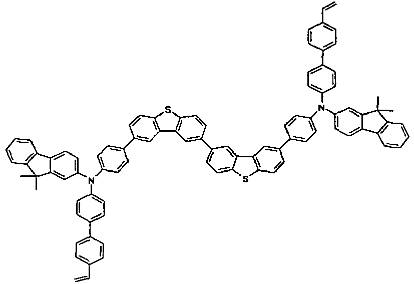 |
| (11) | (12) |
| 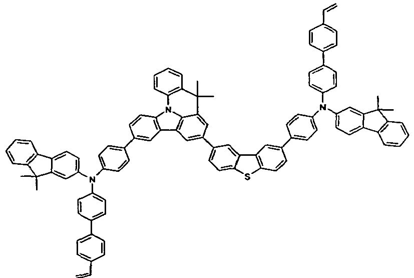 | 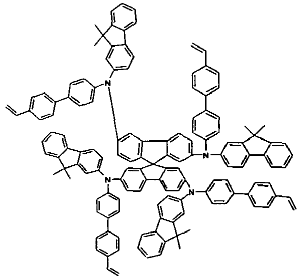 |
| (13) | (14) |
| 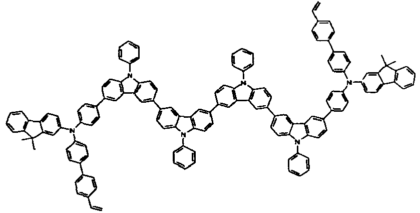 | 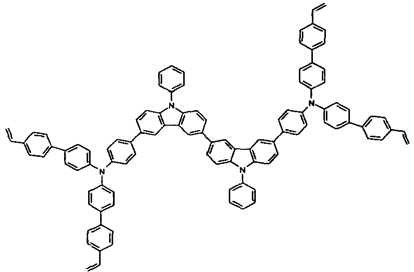 |
| (15) | (16) |
| 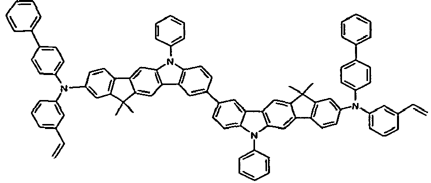 | 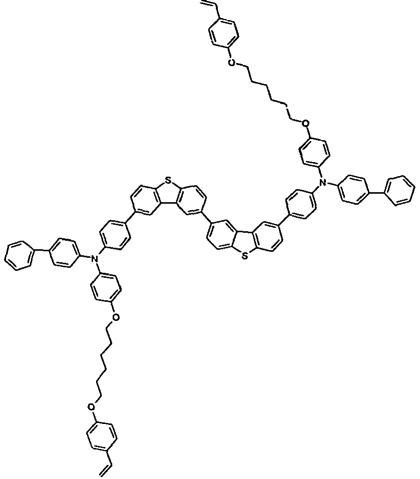 |
| (17) | (18) |
| 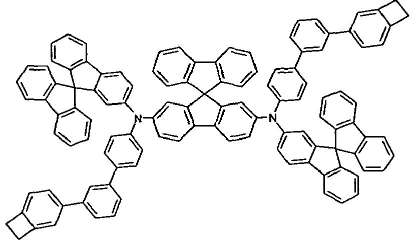 | 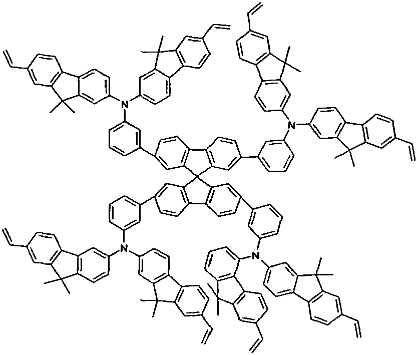 |
| (19) | (20) |
| 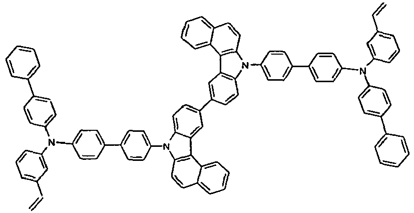 | 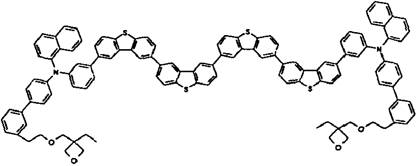 |
| (21) | (22) |
| 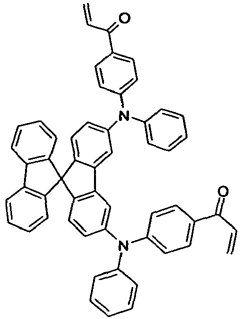 | 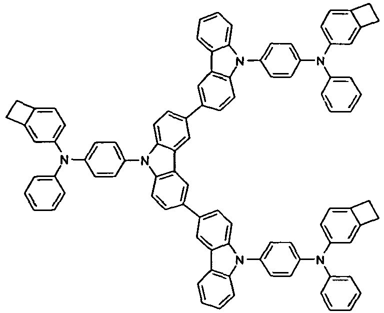 |
| (23) | (24) |
| 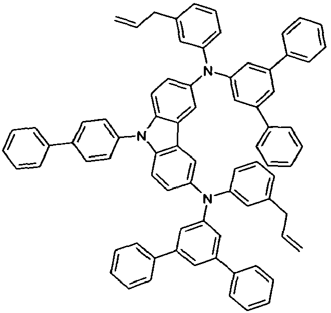 | 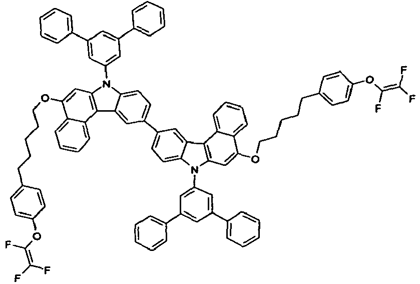 |
| (25) | (26) |
| 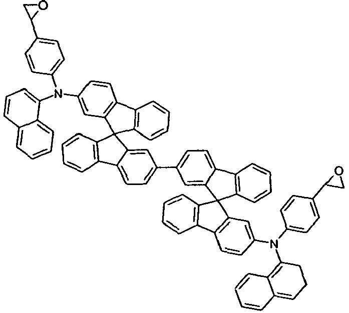 | 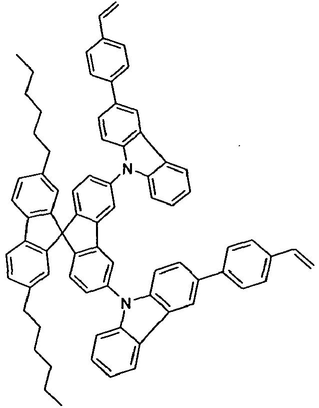 |
| (27) | (28) |
| 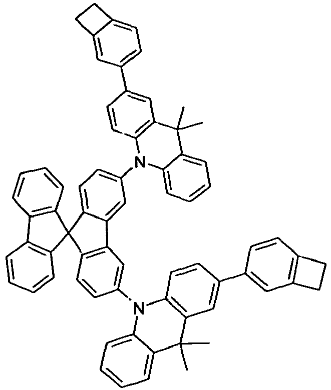 | 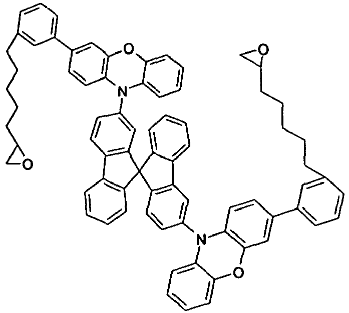 |
| (29) | (30) |
| 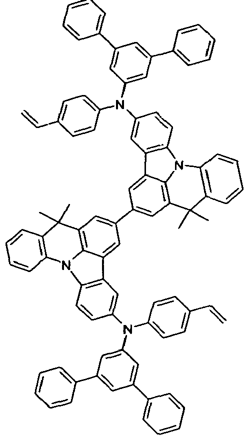 | 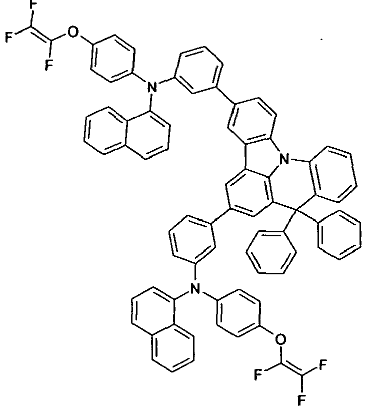 |
| (31) | (32 |
| 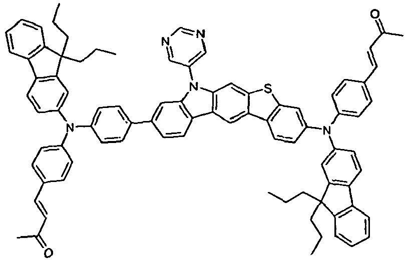 | 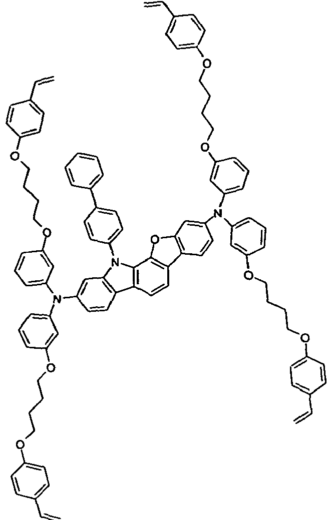 |
| (33) | (34) |
| 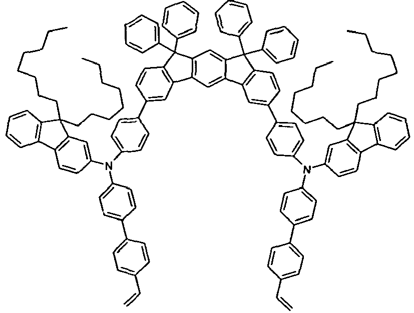 | 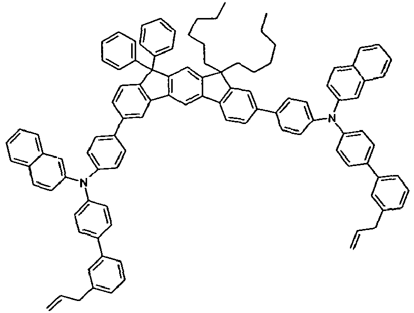 |
| (35) | (36) |
| 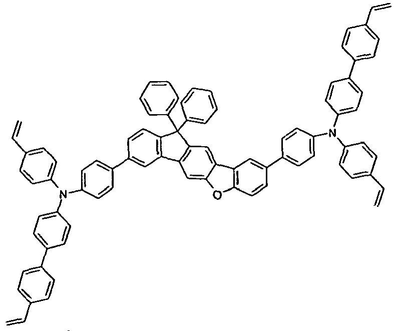 | 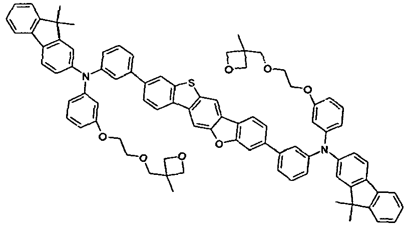 |
| (37) | (38) |
| 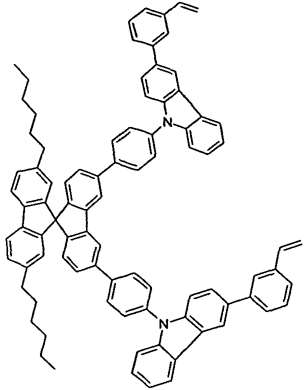 | 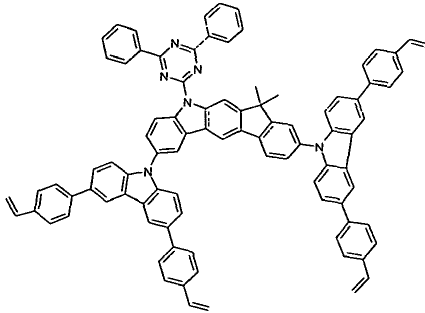 |
| (39) | (40) |
| 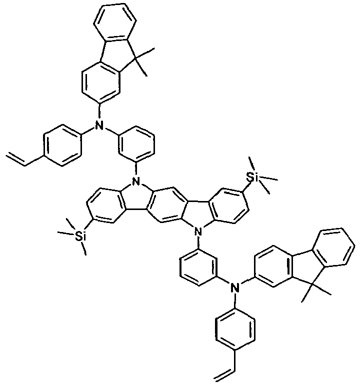 | 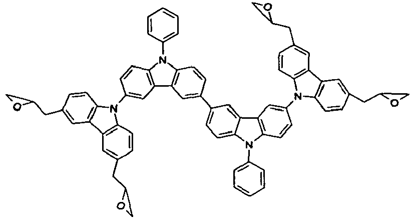 |
| (41) | (42) |
| 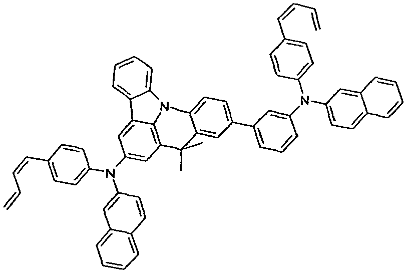 | 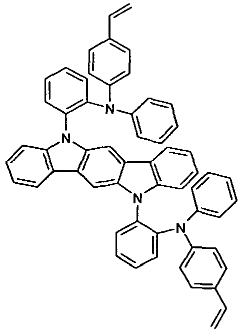 |
| (43) | (44) |
| 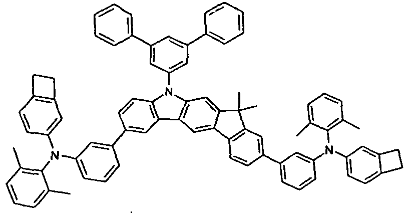 | 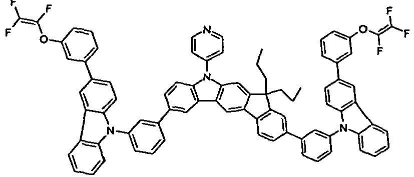 |
| (45) | (46) |
| 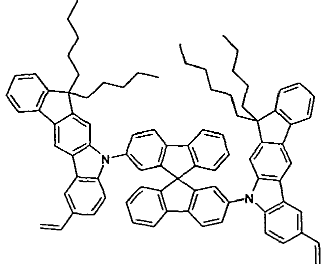 | 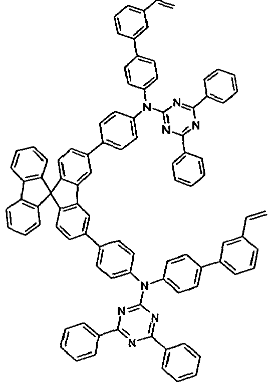 |
| (47) | (48) |
| 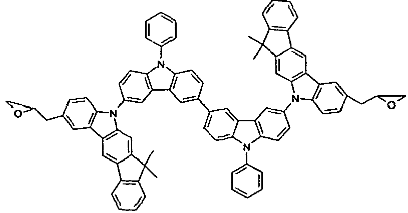 | 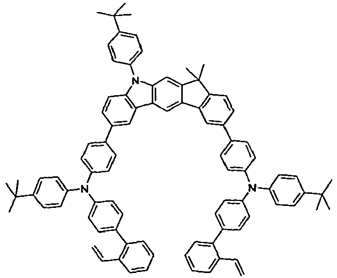 |
| (49) | (50) |
| 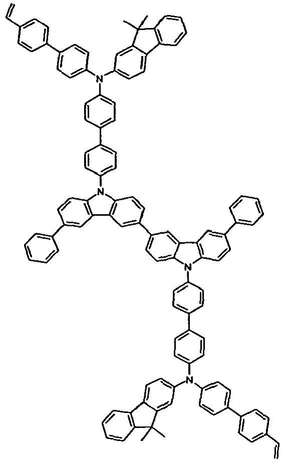 | 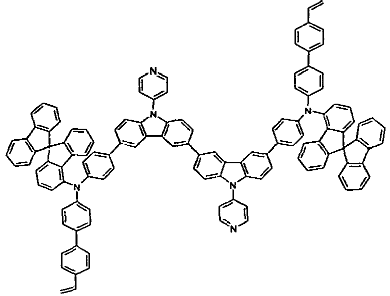 |
| (51) | (52) |
| 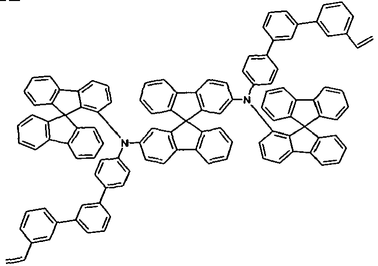 | 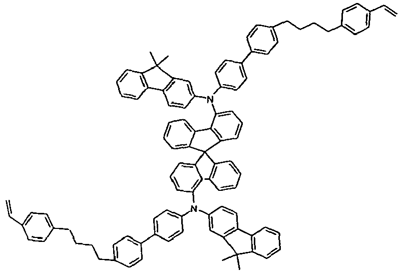 |
| (53) | (54) |
| 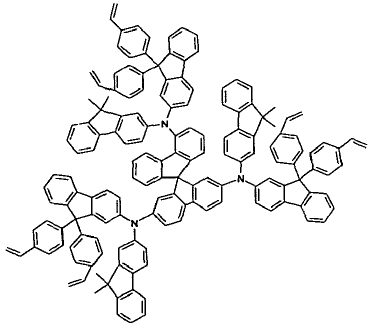 | 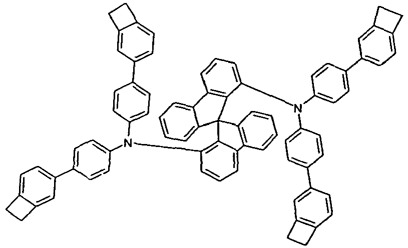 |
| (55) | (56) |
| 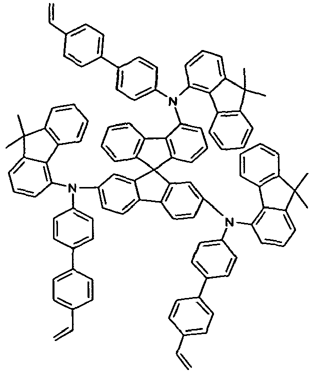 | 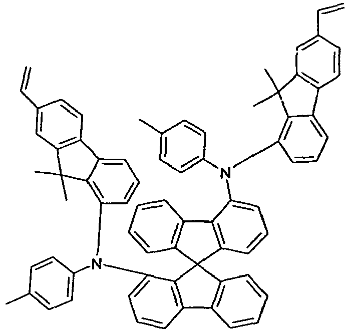 |
| (57) | (58) |
| 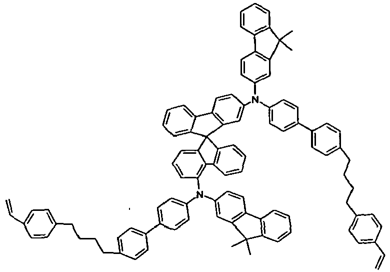 | 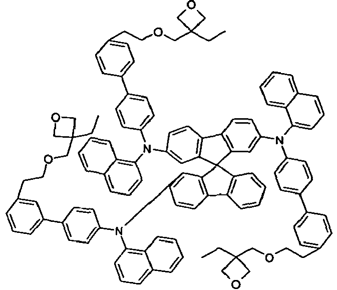 |
| (59) | (60) |

Ein weiterer Gegenstand der vorliegenden Erfindung ist eine vernetzte Verbindung, die durch Vernetzung der Gruppen Q der Verbindung gemäß Formel (1) erhältlich ist. Eine vernetzte Verbindung im Sinne der vorliegenden Erfindung ist eine Verbindung, die erhältlich ist aus der Verbindung gemäß Formel (1) durch Durchführung der Reaktion der vernetzbaren Gruppe Q.

Die vernetzbare Verbindung gemäß Formel (1) kann durch Beschichtung aus Lösung auf ein entsprechendes Trägersubstrat (Glas, Polymer etc.) bzw. eine bereits davor abgeschiedene Schicht aufgebracht werden und entweder vor oder nach Entfernen des Lösemittels oder während des Entfernens des Lösemittels vernetzt werden.

Gegenstand der vorliegenden Erfindung ist somit auch eine Schicht, enthaltend ein oder mehrere erfindungsgemäße Verbindungen wie oben definiert bzw. enthaltend ein oder mehrere Verbindungen, die durch Vernetzung der erfindungsgemäßen Verbindungen erhalten wurden.

Ein weiterer Gegenstand der vorliegenden Erfindung ist ein Verfahren zur Herstellung einer vernetzten Schicht durch Aufbringen und Vernetzung einer Verbindung gemäß Formel (1). Dabei kann die Verbindung gemäß Formel (1) als Reinsubstanz vernetzt werden, oder sie kann als Mischung mit mindestens einer anderen vernetzbaren oder polymerisierbaren Verbindung aufgebracht werden und zusammen mit dieser vernetzt werden. In einer bevorzugten Ausführungsform der Erfindung wird die Verbindung gemäß Formel (1) als Reinsubstanz vernetzt.

Die Erzeugung der Schicht kann beispielsweise durch Beschichtung aus Lösung, vorzugsweise durch Tiefdruck (Gravure Printing), Tintenstrahldruck (Ink-Jet Printing), Nozzle Printing, Flexodruck, Dye Coating, Siebdruck oder Spin-Coating erfolgen. Nach dem Aufbringen einer Schicht der Verbindung gemäß Formel (1) und gegebenenfalls Entfernen des Lösungsmittels kann die Verbindung vernetzt werden. Die Vernetzung erfolgt vorzugsweise strahlungsinduziert (z. B. mit UV-Licht, sichtbarem Licht, Mikrowellen, Elektronenstrahlen) oder thermisch, insbesondere thermisch.

Ein weiterer Gegenstand der vorliegenden Erfindung ist eine Formulierung, enthaltend mindestens eine Verbindung gemäß Formel (1) und ein oder mehrere Lösungsmittel. Wie solche Formulierungen hergestellt werden können, ist dem Fachmann bekannt und z. B. in der WO 2002/072714, der WO 2003/019694 und der darin zitierten Literatur beschrieben.

Geeignete und bevorzugte Lösungsmittel sind beispielsweise Toluol, Anisol, Xylole, Methylbenzoat, Dimethylanisole, Mesitylene, Tetralin, Veratrol, Tetrahydrofuran und Chlorbenzol sowie Gemische derselben.

Gegenstand der voliegenden Erfindung ist auch die Verwendung der erfindungsgemäßen Verbindungen sowie die daraus erhaltenen vernetzten Verbindungen in einer elektronischen Vorrichtung.

Nochmals ein weiterer Gegenstand der vorliegenden Erfindung ist eine elektronische Vorrichtung enthaltend ein oder mehrere Verbindungen gemäß Formel (1) bzw. ein oder mehrere vernetzte Verbindungen, die durch Vernetzung der Verbindung gemäß Formel (1) erhalten wurden.

Die elektronische Vorrichtung ist vorzugsweise eine organische Elektrolumineszenzvorrichtung (OLED), eine organische integrierte Schaltung (O-IC), ein organischer Feld-Effekt-Transistor (O-FT), ein organischer Dünnfilmtransistor (O-TFT), ein organischer, lichtemittierender Transistor (O-LET), eine organische Solarzelle (O-SC), ein organischer, optischer Detektor, ein organischer Fotorezeptor, ein organisches Feld-Quench-Device (O-FQD), eine lichtemittierende elektrochemische Zelle (LEC) oder eine organische Laserdiode (O-Laser), vorzugsweise eine organische Elektrolumineszenzvorrichtung (OLED).

In einer weiteren Ausführungsform der vorliegenden Erfindung umfasst die Vorrichtung mehrere Schichten. Die erfindungsgemäße Verbindung gemäß Formel (1) bzw. die daraus erhaltene vernetzte Verbindung kann dabei in einer Lochtransport-, Lochinjektions-, Emitter-, Elektronentransport-, Elektroneninjektions-, Ladungsblockier- und/oder Ladungserzeugungsschicht vorliegen.

Die erfindungsgemäße organische Elektrolumineszenzvorrichtung enthält Kathode, Anode und mindestens eine emittierende Schicht. Außer diesen Schichten kann die organische Elektrolumineszenzvorrichtung noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten und/oder Ladungserzeugungsschichten (Charge-Generation Layers). Ebenso können zwischen zwei emittierende Schichten Zwischenschichten eingebracht sein, welche beispielsweise eine Exzitonen-blockierende Funktion aufweisen. Es sei aber darauf hingewiesen, dass nicht notwendigerweise jede dieser Schichten vorhanden sein muss. Möglich ist auch, dass mehrere OLEDs übereinander angeordnet werden, wodurch eine weitere Effizienzsteigerung hinsichtlich der Lichtausbeute erreicht werden kann. Zur Verbesserung der Lichtauskopplung kann die letzte organische Schicht auf der Lichtaustrittseite bei OLEDs beispielsweise auch als Nanoschaum oder als anderes Material mit einem geringen Brechungsindex ausgeführt sein, wodurch der Anteil der Totalreflexion verringert wird.

Es ist auch möglich, dass die erfindungsgemäße organische Elektrolumineszenzvorrichtung mehrere emittierende Schichten enthält. Wenn mehrere Emissionsschichten vorhanden sind, weisen diese vorzugsweise insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können.

Die Vorrichtung kann Schichten enthalten, welche aus niedermolekularen Verbindungen aufgebaut sind. Diese können durch Aufdampfen der Verbindungen im Hochvakuum oder durch Aufbringen aus Lösung erzeugt werden. Die Vorrichtung kann ebenfalls Schichten enthalten, welche aus oligomeren, polymeren oder dendritischen Verbindungen aufgebaut sind. Diese werden insbesondere durch Aufbringen aus Lösung erzeugt.

Vorzugsweise weist die erfindungsgemäße organische Elektrolumineszenzvorrichtung den folgenden Aufbau auf: Anode / optional Schicht aus einem leitfähigen Polymer / eine oder mehrere vernetzte Schichten, erhältlich durch Vernetzung der Verbindung der Formel (1) / Emissionsschicht und Kathode.

In einer bevorzugten Ausführungsform der vorliegenden Erfindung werden die erfindungsgemäßen Verbindungen sowie die daraus erhaltenen vernetzten Verbindungen in einer Lochtransportschicht bzw. in einer Lochinjektionsschicht verwendet. Diese Schicht wird als Zwischenschicht zwischen der Anode bzw. dem leitfähigen Polymer und der emittierenden Schicht verwendet. Eine Lochinjektionsschicht im Sinne der vorliegenden Erfindung bezeichnet eine Schicht, die direkt an die Anode angrenzt. Eine Lochtransportschicht im Sinne der vorliegenden Erfindung bezeichnet eine Schicht, die zwischen einer Lochinjektionsschicht und einer emittierenden Schicht angeordnet ist.

Dabei kann die vernetzte Lochtransportschicht bzw. die vernetzte Lochinjektionsschicht auch dotiert sein, insbesondere mit Elektronenakzeptor-Verbindungen, beispielsweise mit F₄-TCNQ oder mit Verbindungen, wie sie in der EP 1476881 oder der EP 1596445 beschrieben werden.

Insbesondere für Beleuchtungsanwendungen ist es bevorzugt, wenn eine p-dotierte Schicht der Verbindung der Formel (1) direkt auf die Anode aufgebracht und dort vernetzt wird. Hier ist eine zusätzliche Schicht aus einem leitfähigen Polymer nicht erforderlich, d. h. die vernetzte Schicht, die durch Vernetzung der Verbindung der Formel (1) erhalten wird, dient als Ersatz für das dotierte leitfähige Polymer. Die Schichtdicke dieser Schicht beträgt vorzugsweise zwischen 10 und 400 nm, besonders bevorzugt zwischen 50 und 350 nm.

Weiterhin bevorzugt werden die erfindungsgemäßen Verbindungen gemäß Formel (1) sowie die daraus erhaltenen erfindungsgemäßen vernetzten Verbindungen in einer Lochtransportschicht verwendet, wobei diese Lochtransportschicht auf eine Schicht eines leitfähigen Polymers aufgebracht ist. Als leitfähiges Polymer eignen sich alle Materialien, wie sie der Fachmann üblicherweise für diese Schicht verwendet, beispielsweise PEDOT/PSS, dotiertes PANI oder dotierte Oligoaniline. Die Schichtdicke der erfindungsgemäßen Lochtransportschicht liegt üblicherweise im Bereich von 10 bis 400 nm, vorzugsweise im Bereich von 50 bis 200 nm.

In den weiteren Schichten können alle Materialien verwendet werden, wie sie üblicherweise in organischen Elektrolumineszenzvorrichtungen verwendet werden und wie sie dem Fachmann bekannt sind.

Wenn als emittierende Schicht eine phosphoreszierende Schicht verwendet wird, so besteht diese vorzugsweise aus niedermolekularen Verbindungen, die aus Lösung aufgebracht werden.

Wenn als emittierende Schicht eine blaue fluoreszierende Schicht verendet wird, so besteht diese vorzugsweise aus niedermolekularen Verbindungen, die entweder aus Lösung aufgebracht oder im Vakuum aufgedampft werden.

Insbesondere in den oben genannten Fällen für phosphoreszierende oder blau fluoreszierende Emitterschichten, aber auch in anderen Elektrolumineszenzvorrichtungen ist es bevorzugt, wenn die organische Elektrolumineszenzvorrichtung eine Elektronentransportschicht enthält. Diese wird vorzugsweise auf die emittierende Schicht aufgedampft. Geeignete Materialien für die Elektronentransportschicht sind Benzimidazolderivate, Triazinderivate und/oder Hydroxychinolin-Komplexe, wie z. B. LiQ (Lithiumchinolinat).

Bevorzugt ist eine organische Elektrolumineszenzvorrichtung, die dadurch gekennzeichnet ist, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Insbesondere bevorzugt wird die Verbindung gemäß Formel (1) aus Lösung aufgebracht.

Weiterhin bevorzugt ist eine organische Elektrolumineszenzvorrichtung, wobei eine oder mehrere Schichten mit einem Sublimationsverfahren beschichtet werden. Dabei werden die Materialien in Vakuum-Sublimationsanlagen bei einem Druck kleiner 10⁻⁵ mbar, vorzugsweise kleiner 10⁻⁶ mbar, besonders bevorzugt kleiner 10⁻⁷ mbar aufgedampft.

Bevorzugt ist ebenfalls eine organische Elektrolumineszenzvorrichtung, die dadurch gekennzeichnet ist, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht.

Es ist auch möglich, die organische Elektrolumineszenzvorrichtung als Hybrid-Device herzustellen, indem beispielsweise ein oder mehrere Schichten aus Lösung aufgebracht werden und eine oder mehrere weitere Schichten im Vakuum aufgedampft werden. So kann beispielsweise die Schicht enthaltend die Verbindung gemäß Formel (1) aus Lösung aufgebracht werden und die emittierende Schicht kann aufgedampft werden. Ebenso kann die Schicht enthaltend die Verbindung gemäß Formel (1) sowie die emittierende Schicht aus Lösung aufgebracht werden und die Elektronentransportschicht kann aufgedampft werden.

Die Vorrichtung enthält gewöhnlich eine Kathode und eine Anode (Elektroden). Die Elektroden werden im Sinne der vorliegenden Erfindung so gewählt, dass ihr Potential möglichst gut mit dem Potential der angrenzenden organischen Schicht übereinstimmt, um eine möglichst effiziente Elektronen- bzw. Lochinjektion zu gewährleisten.

Als Kathode sind Metallkomplexe, Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Ba/Al, Mg/Ag, Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide in Frage (z. B. LiF, Li₂O, BaF₂, MgO, NaF, etc.). Die Schichtdicke dieser Schicht liegt vorzugsweise zwischen 1 und 10 nm, besonders bevorzugt zwischen 2 und 8 nm.

Als Anode sind Materialien mit hoher Austrittsarbeit bevorzugt. Vorzugsweise weist die Anode ein Potential größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (O-SC) oder die Auskopplung von Licht (OLED/PLED, O-LASER) zu ermöglichen. Ein bevorzugter Aufbau verwendet eine transparente oder teiltransparente Anode. Bevorzugte Anodenmaterialien sind hier leitfähige, gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere, wie z. B. Poly(ethylendioxythiophen) (PEDOT) und Polyanilin (PANI).

Als Schicht direkt auf der Anode werden vorzugsweise leitfähige dotierte Polymere, wie z. B. jeweils dotiertes Poly(ethylendioxythiophen) (PEDOT), Polyanilin (PANI) oder Oligoanilin, verwendet, wenn hierfür nicht eine erfindungsgemäße Schicht verwendet wird.

Die Vorrichtung wird je nach Anwendung entsprechend strukturiert, kontaktiert und schließlich hermetisch versiegelt, da sich die Lebensdauer derartiger Vorrichtungen bei Anwesenheit von Wasser und/oder Luft drastisch verkürzt.

Die Erfindung wird durch die nachfolgenden Beispiele näher erläutert, ohne sie dadurch einschränken zu wollen. Der Fachmann kann aus den Schilderungen ohne erfinderisches Zutun weitere erfindungsgemäße elektronische Vorrichtungen herstellen und somit die Erfindung im gesamten beanspruchten Bereich ausführen.

### Beispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Die Angaben in eckigen Klammern zu literaturbekannten chemischen Verbindungen beziehen sich auf die CAS-Nummer.

### Beispiel 1: Synthese von Verbindung 3

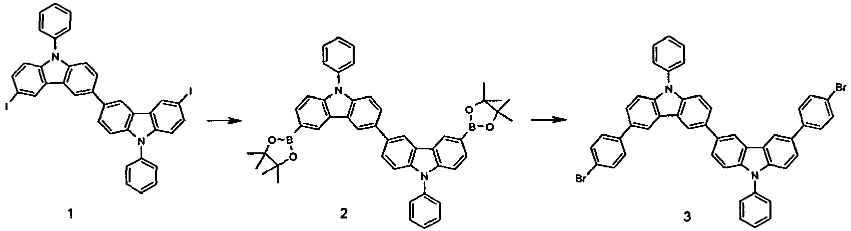

### a) Synthese von Verbindung 2

In einem ausgeheizten Kolben werden 100 g (136 mmol; 1.0 äq.) Verbindung **1** [57102-64-4], 135 g (0.53 mol; 3.9 äq.) Bis(pinakolato)diboran und 160 g (1.63 mol; 12 äq.) Kaliumacetat in 1.1 l entgastem Dioxan suspendiert. Die Suspension wird 30 Minuten entgast und daraufhin 4.40 g (5.44 mmol; 0.04 äq.) PdCl₂(dppf)·CH₂Cl₂ als Katalysator zugegeben. Die Reaktionsmischung wird 72 Stunden unter Rückfluss erhitzt. Der braune Niederschlag wird filtriert und mit 1 l Wasser gewaschen. Der erhaltene Feststoff wird mit Dichlormethan versetzt und mit Wasser extrahiert. Die organischen Phasen werden vereint, über Na₂SO₄ getrocknet, filtriert und die Lösungsmittel im Vakuum entfernt. Es werden 68.0 mmol (50%, HPLC Reinheit 95%) eines braunen Feststoffs erhalten. Die erhaltene Verbindung **2** wird zweimal aus o-Xylol umkristallisiert und dann über Aluminiumoxid mit o-Xylol heißextrahiert. Es wird ein hell-beiger Feststoff erhalten, welcher aus DMF umkristallisiert wird. Anschließend wird mehrmals aus Anisol umkristallisiert, bis eine HPLC Reinheit von 99.2% erreicht wird. Die Ausbeute beträgt 22.9 g (31.2 mmol), entsprechend 22.9% der Theorie.

### b) Synthese von Verbindung 3

22.9 g (31.2 mmol; 1.0 äq.) Verbindung **2**, 22.1 g (78.1 mmol; 2.5 äq.) 1-Brom-4-iodbenzol und 10.6 g Natriumcarbonat (99.9 mmol; 3.2 äq.) werden in einer Mischung aus entgastem Wasser/Toluol/Dioxan (185/160/90 ml) suspendiert. Die Suspension wird 30 Minuten entgast und daraufhin 721 mg (0.625 mmol;0.02 äq.) Pd(PPh₃)₄ zugegeben. Anschließend wird die Reaktionsmischung 27 Stunden unter Rückfluss erhitzt und nach beendeter Reaktion filtriert. Der weiß-beige Feststoff wird jeweils mit Wasser, Toluol und abschließend mit Heptan gewaschen und der Rückstand im Vakuumtrockenschrank bei 85°C getrocknet. Das erhaltene Rohprodukt (24.5 mmol) wird aus DMF und anschließend aus Chlorbenzol umkristallisiert, bis eine HPLC-Reinheit von 99 % erreicht ist. Die Ausbeute beträgt 7.95 g (10 mmol), entsprechend 32 % der Theorie.

### Beispiel 2: Synthese von Verbindung 8

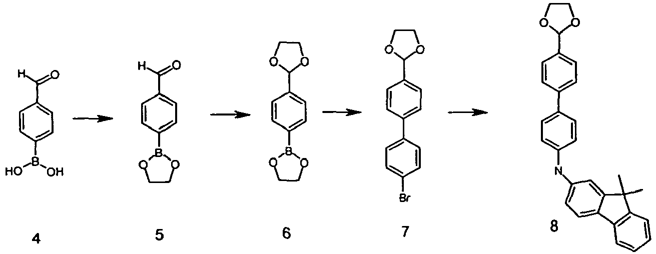

### a) Synthese von Verbindung 5

5.00 g (33.0 mmol; 1.0 äq.) 4-Formylphenylboronsäure (Verbindung **4**) werden in 30 ml Toluol suspendiert. Daraufhin werden 1.9 ml Ethylenglykol (33.0 mmol; 1.0 äq.) zugegeben. Die Reaktionsmischung wird für 2.5 Stunden unter Rückfluss gerührt und das erhaltene Wasser kontinuierlich mit einem Wasserabscheider entfernt. Die Reaktionsmischung wird auf Raumtemperatur abgekühlt und die Lösungsmittel im Vakuum entfernt. Die Ausbeute beträgt 5.86 g (33.0 mmol), entsprechend 100 % der Theorie.

### b) Synthese von Verbindung 6

5.86 g (33.0 mmol; 1.0 äq.) Verbindung **5** werden in 150 ml Toluol mit 10.0 g Aluminiumoxid (sauer) suspendiert. Daraufhin werden 11.3 ml Ethylenglykol (200 mmol; 6.0 äq.) zugegeben. Die Reaktionsmischung wird 24 Stunden unter Rückfluss gerührt. Nach beendeter Reaktion wird der Ansatz filtriert und das Aluminiumoxid mit Dichlormethan gewaschen. Die organischen Phasen werden vereinigt und die Lösungsmittel im Vakuum entfernt. Die Ausbeute beträgt 7.00 g (32.0 mmol), entsprechend 97 % der Theorie.

### c) Synthese von Verbindung 7

14.5 g 2-(4-[1,3]-Dioxolan-2-yl-phenyl)-[1,3,2]dioxaborolan (Verbindung **6**) (66.0 mmol; 1.0 äq.), 18.7 g 1-Brom-4-iodbenzol (66.0 mmol; 1.0 äq.) und 18.2 g Natriumcarbonat (132 mmol; 2.0 äq.) werden in einer Mischung aus entgastem Wasser/Toluol (85/220 ml) suspendiert. Die Suspension wird 30 Minuten entgast und anschließend 763 mg (0.66 mmol; 0.01 äq.) Pd(PPh₃)₄ zugegeben. Die Reaktionsmischung wird 24 Stunden unter Rückfluss gerührt und nach beendeter Reaktion mit 75 ml einer Hydrogencarbonat-Lösung (0.6 M; pH = 8.2) gewaschen. Die organische Phase wird über Na₂SO₄ getrocknet, filtriert und die Lösungsmittel im Vakuum entfernt. Der erhaltene, gelbe Feststoff wird 3 Stunden bei 60°C in 25 ml Heptan gerührt. Anschließend wird die Suspension filtriert, der Feststoff in Dichlormethan gelöst und über Celite filtriert. Nach Entfernung des Lösungsmittels im Vakuum beträgt die Ausbeute 13.3 g (43.6 mmol), entsprechend 66 % der Theorie.

### d) Synthese von Verbindung 8

3.80 g 2-(4'-Brom-biphenyl-4-yl)-[1,3]dioxolan (Verbindung **7**) (12.0 mmol; 1.0 äq.), 2.60 g 9,9-Dimethyl-9*H*-fluoren-2-ylamin (12.0 mmol; 1.0 äq.) und 1.80 g Natrium-*tert*-butylat (19.0 mmol; 1.5 äq.) werden in 30 ml entgastem Toluol suspendiert und 30 Minuten entgast. 28.0 mg (0.031 mmol; 0.026 äq.) Pd(dba)₃ und 39.0 mg (0.062 mmol; 0.052 äq.) BINAP werden in 10 ml entgastem Toluol in einem 20 ml-Bördelrandgläschen vorgelegt. Die Katalysator-Lösung wird 15 Minuten entgast und zur Reaktionsmischung gegeben. Der Ansatz wird 5 Stunden unter Rückfluss erhitzt. Nach beendeter Reaktion werden 50 ml Toluol zugegeben und die Reaktionsmischung mit Wasser gewaschen. Die organische Phase wird über Na₂SO₄ getrocknet, filtriert und die Lösungsmittel im Vakuum entfernt. Der orange-braune Feststoff wird aus Heptan/Toluol (4:3) umkristallisiert. Die Ausbeute beträgt 2.90 g (6.70 mmol) eines gelben Feststoffs, entsprechend 54 % der Theorie.

### Beispiel 3: Synthese von Verbindung 9

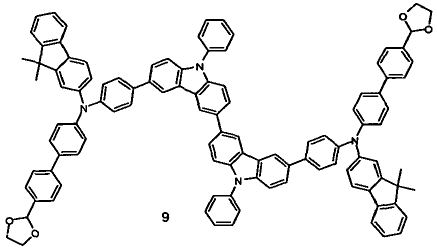

7.00 g Verbindung **3** (8.81 mmol; 1 äq), 8.40 g (19.4 mmol; 2.2 äq.) Verbindung **8** und 2.54 g Natrium-*tert*-butylat (26.4 mmol; 3 äq.) werden in 200 ml entgastem Toluol suspendiert und 20 Minuten entgast. Anschließend werden 0.264 ml Tri-*tert*-butylphosphin 1M in Toluol (0.264 mmol; 0.03 äq.) und 19.8 mg Pd(OAc)₂ (0.088 mmol; 0.001 äq.) in 2 ml entgastem Toluol zu der Reaktionsmischung gegeben. Der Ansatz wird 30 Stunden bei 110°C erhitzt und nach beendeter Reaktion 200 ml Ethylacetat und 120 ml gesättigte NaHCO₃-Lösung zugegeben. Die wässrige Phase wird mit 3 x 200 ml Ethylacetat extrahiert. Die organische Phase wird über Na₂SO₄ getrocknet, filtriert und die Lösungsmittel im Vakuum entfernt. Der Feststoff wird in warmem Toluol gerührt, abgekühlt und filtriert. Die Ausbeute beträgt 7.13 g (4.75 mmol) eines gelben Feststoffs, entsprechend 54 % der Theorie.

### Beispiel 4: Synthese von Verbindung 10

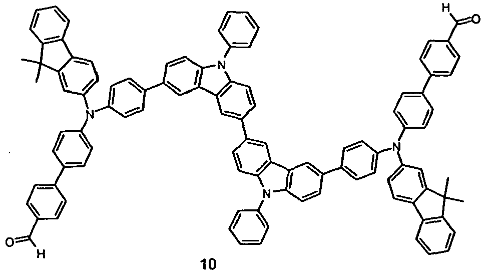

6.50 g Verbindung **9** (4.30 mmol; 1 äq), gelöst in 200 ml Chloroform, und 100 ml einer 7 %igen wässrigen HCI-Lösung werden 1 Stunde bei 75°C erhitzt. Nach beendeter Reaktion wird die organische Phase mit 3 x 200 ml Wasser gewaschen, getrocknet über Na₂SO₄ und filtriert. Die Lösungsmittel werden im Vakuum entfernt und der gelbe Rückstand wird in einer Mischung Chloroform/Heptan und dann Tetrahydrofuran/Acetonitril umkristallisiert. Die Ausbeute beträgt 1.95 g (1.38 mmol) eines gelben Feststoffs, entsprechend 32 % der Theorie.

### Beispiel 5: Synthese der erfindungsgemäßen Verbindung 11

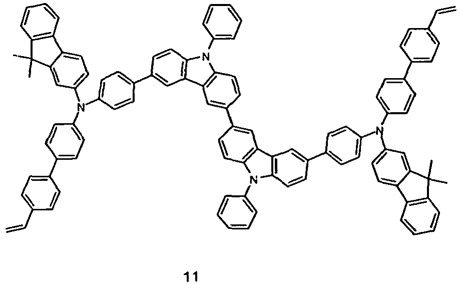

0.91 g (2.6 mmol; 4 äq.) Methyltriphenylphosphoniumbromid werden in 15 ml getrocknetem THF unter Argon bei 0°C suspendiert. Das Reaktionsgemisch wird portionsweise mit 0.29 g (2.6 mmol; 4 äq.) Kalium-*tert-*butylat versetzt und 40 Minuten unter Eiskühlung gerührt. 1.0 g (0.7 mmol; 1.0 äq.) Verbindung **10** werden in 50ml THF gelöst und zugegeben. Das Eisbad wird nach 30 Minuten entfernt und das Reaktionsgemisch wird 6 Tage bei Raumtemperatur gerührt. 60 ml Wasser und 120 ml Ethylacetat werden zugegeben, die organische Phase wird mit 2 x 50 ml Wasser gewaschen, getrocknet über Na₂SO₄ und am Rotationsverdampfer eingeengt. Der Rückstand wird mit 2 x 20 ml Methanol gewaschen, filtriert, aus Chloroform/Methanol umkristallisiert und mit Dichlormethan/Heptan (1/3) über Kieselgel chromatographiert. Die Ausbeute beträgt 0.43 g (0.3 mmol) eines hellgelben Feststoffs, entsprechend 43 % der Theorie.

### Beispiel 6: Synthese von Verbindung 13 (kein Teil der vorliegenden Erfindung, weil n=1)

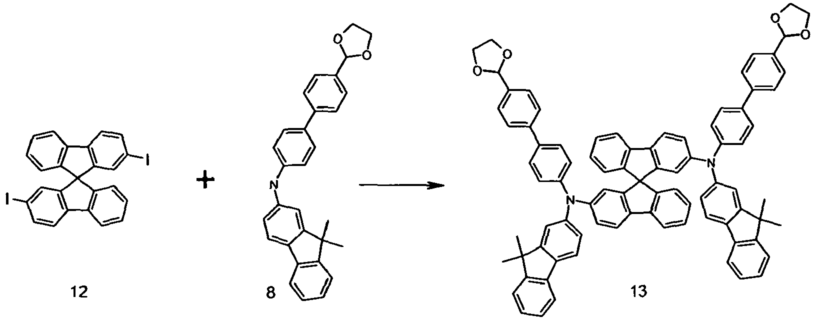

2.00 g (3.5 mmol; 1 äq.) Verbindung **12** [790674-48-5], 3.35 g (7.7 mmol; 2.2 äq.) Verbindung **8** und 1.01 g Natrium-*tert*-butylat (10.5 mmol; 3 äq.) werden in 30 ml entgastem Toluol gelöst und 20 Minuten entgast. Anschließend werden 0.105 ml Tri-tert-butylphosphin 1M in Toluol (0.105 mmol; 0.03 äq.) und 7.8 mg Pd(OAc)₂ (0.035 mmol; 0.001 äq.) in 5 ml entgastem Toluol zu der Reaktionsmischung gegeben. Der Ansatz wird 2.5 Stunden bei 110°C erhitzt. Nach beendeter Reaktion werden 200 ml Ethylacetat zugegeben und die Reaktionsmischung über Celite filtriert. Die organische Phase wird mit 2 x 60 ml gesättigter NaHCO₃ Lösung gewaschen, über Na₂SO₄ getrocknet, filtriert und die Lösungsmittel im Vakuum entfernt. Der Feststoff wird in warmem Heptan /Toluol gerührt, abgekühlt und filtriert. Die Ausbeute beträgt 3.40 g (2.88 mmol) eines gelben Feststoffs, entsprechend 82 % der Theorie.

### Beispiel 7: Synthese von Verbindung 14 (kein Teil der vorliegenden Erfindung, weil n = 1)

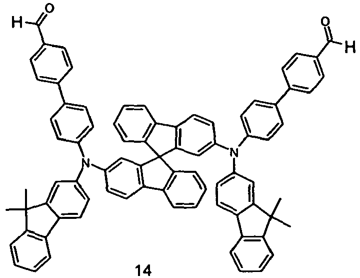

3.50 g Verbindung **13** (2.97 mmol; 1 äq), gelöst in 40 ml Chloroform, und 10 ml einer 3.5 %igen wässrigen HCI-Lösung werden über Nacht auf 75°C erhitzt. Nach beendeter Reaktion wird die organische Phase mit 30 ml gesättigter Lösung NaHCO₃ und 2 x 200 ml Wasser gewaschen, über Na₂SO₄ getrocknet und filtriert. Die Lösungsmittel werden im Vakuum entfernt und der gelbe Rückstand wird aus Toluol/Heptan und aus Chloroform/Heptan umkristallisiert. Die Ausbeute beträgt 2.9 g (2.66 mmol) eines gelben Feststoffs, entsprechend 90 % der Theorie.

### Beispiel 8: Synthese der erfindungsgemäßen Verbindung 15 (kein Teil der vorliegenden Erfindung, weil n = 1)

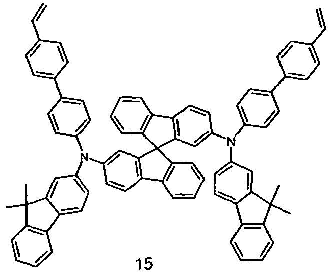

3.00 g (9.2 mmol; 4 äq.) Methyltriphenylphosphoniumbromid werden in 100 ml getrocknetem THF unter Argon bei 0°C suspendiert. Das Reaktionsgemisch wird portionsweise mit 1.03 g (9.2 mmol; 4 äq.) Kaliumtert-butylat versetzt und 30 Minuten unter Eiskühlung gerührt. 2.5 g (2.3 mmol; 1.0 äq.) Verbindung **14** wird in 100 ml THF gelöst und zugegeben. Das Eisbad wird nach 30 Minuten entfernt und das Reaktionsgemisch wird 1 Stunde bei Raumtemperatur gerührt. 120 ml Wasser und 200 ml DCM werden zugegeben, die organische Phase wird mit 2 x 50 ml Wasser gewaschen, über Na₂SO₄ getrocknet und am Rotationsverdampfer eingeengt. Der Rückstand wird mit 2 x 20 ml Methanol gewaschen, filtriert und aus Chloroform/Methanol und DMF/Methanol umkristallisiert. Die Ausbeute beträgt 1.75 g (1.6 mmol) eines hellgelben Feststoffs, entsprechend 70 % der Theorie.

### Beispiel 9: Elektrolumineszenzvorrichtungen (Beispiel, das keinen Teil der vorliegenden Erfindung bildet)

Die Herstellung einer aus Lösung prozessierten organischen Leuchtdiode (OLED) ist in der Literatur bereits vielfach beschrieben (z.B. in der WO 2004/037887). Um die vorliegende Erfindung beispielhaft zu erläutern, werden OLEDs mit dem Material 1 sowie dem Vergleichsmaterial 1 durch Spincoating hergestellt.

Eine typische OLED hat den im Folgenden dargestellten Aufbau, wobei das erfindungsgemäße Material 1 die Funktion einer Lochtransportschicht (Hole-Transport-Layer; HTL) erfüllt.

| | | |
|---|---|---|
| 3 nm / 100 nm | **Kathode** | Ba/Al |
| 80 nm | **EML** | EML |
| 20 nm | **HTL** | Material 1 bzw. Vergleich 1 |
| 80 nm | **Buffer** | PEDOT:PSS |
| | **Anode** | ITO |

Ein mit Indium-Zinn-Oxid (ITO) beschichtetes Glas stellt das Substrat dar. Dieses wird im Reinraum mit entionisiertem Wasser und einem Detergens gereinigt und dann durch eine UV/Ozon-Plasmabehandlung aktiviert. Danach wird ebenfalls im Reinraum eine 80 nm dicke Schicht PEDOT:PSS (Baytron P VAI 4083sp von H.C. Starck (mittlerweile Heraeus Clevios), das als wässrige Dispersion geliefert wird) durch Spin-Coating aufgebracht. Die benötigte Spinrate hängt vom Verdünnungsgrad und der spezifischen Spincoater-Geometrie ab (typisch für 80 nm: 4500 rpm). Um Restwasser aus der Schicht zu entfernen, werden die Substrate für 10 Minuten bei 180°C auf einer Heizplatte ausgeheizt. Danach werden in Inertgasatmosphäre (Stickstoff bzw. Argon) zunächst 20 nm der HTL-Schicht aufgebracht. Das Aufbringen erfolgt durch Spincoating aus einer Toluol-Lösung. Anschließend wird diese Schicht bei 180°C 1 Stunde ausgeheizt, um den Vernetzungsprozess zu aktivieren. Anschließend werden dann 80 nm der Emissionsschicht EML ebenfalls aus einer Toluollösung mittels Spincoating aufgebracht. Diese Schicht wird bei 180°C 10 Minuten ausgeheizt.

### Material 1: (kein Teil der vorliegenden Erfindung, weil n = 1)

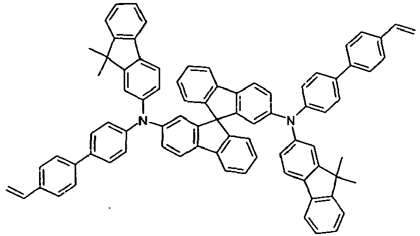

### Vergleich 1 (gemäß WO 2010/097155):

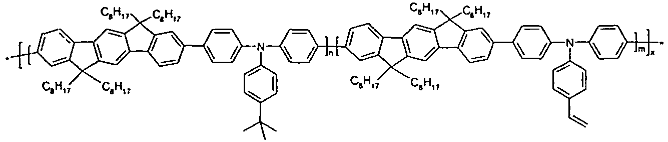

n = 0.9, m = 0.1
Polymerisationsgrad: 250

Als Materialien für die Emissionsschicht (EML) wird eine Mischung von zwei Matrixmaterialien (M1 und M2) und einem phosphoreszierenden grünen Emitter (E1) in einem Mischungsverhältnis (bezogen auf das Gewicht) von M1 : M2 : E1 = 2 : 2 : 1 verwendet.

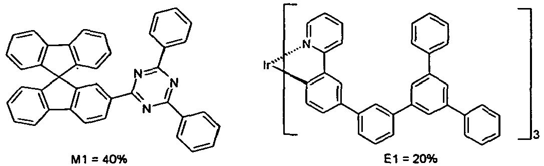

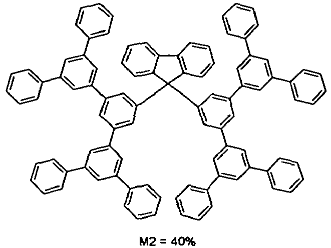

Anschließend wird eine Ba/Al-Kathode durch eine Schattenmaske aufgedampft (hochreine Metalle von Aldrich, besonders Barium 99.99 %); Aufdampfanlagen von Lesker o.a., typischer Vakuumlevel 5 x 10⁻⁶ mbar; Schichtdicke Ba/Al 3 nm/100 nm). Um die Kathode vor Luft und Luftfeuchtigkeit zu schützen, werden die OLEDs abschließend verkapselt und dann charakterisiert.

Die Strom-Spannungs-Leuchtdichte (IVL) Kennlinie der OLEDs wird erhalten, indem die angelegte Spannung schrittweise erhöht (typischerweise von 0 bis max. 10 V in 0.2 V-Schritten) und für jeden Messpunkt der Strom durch die Devices sowie der erhaltene Photostrom einer kalibrierten Photodiode, die sich direkt über der OLED befindet, gemessen wird. Wichtige Kenngrößen sind die gemessene Stromeffizienz [Leuchtdichte / Stromdichte [cd/A]] und Spannung jeweils bei einer Leuchtdichte von 1000 cd/m². Zur Messung des Elektrolumineszenzspektrum und der Farbe der OLEDs wird über eine Lichtleitfaser das emittierte Licht in ein Spektrometer (Ocean Optics) geleitet. Aus dem gemessenen Spektrum können die Farbkoordinaten (CIE: Commission International de l'éclairage, Normalbetrachter von 1931) berechnet werden. Eine weitere wichtige Kenngröße ist die Lebensdauer (LD). Als LD50 wird die Zeit bezeichnet, bei der unter Betrieb bei konstanter Stromdichte die Anfangsleuchtdichte (hier 1000 cd/m²) auf die Hälfte abgefallen ist.

Die Ergebnisse bei Verwendung des erfindungsgemäßen Materials sowie des Vergleichsmaterials sind in Tabelle 1 zusammengefasst.

**Tabelle 1:**

| | Stromeffizienz [cd/A] @ 1000 cd/m² | U[V]@ 1000 cd/m² | CIE [x/y] | LT50 [h] @ 1000 cd/m² |
|---|---|---|---|---|
| Vergleich 1 | 30 | 5.6 | 0.34/0.62 | 41000 |
| Material 1 | 38 | 5.1 | 0.34/0.62 | 53000 |

Das Material 1 zeigt gegenüber dem Vergleichsmaterial, wie aus den Ergebnissen in Tabelle 1 ersichtlich, einen Vorteil in Bezug auf Betriebsspannung, Stromeffizienz und Lebensdauer.

## Patentansprüche

1. Verbindung gemäß Formel (1), 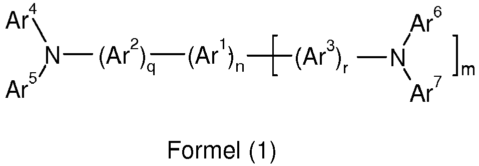 wobei für die verwendeten Symbole und Indices gilt:
Ar¹ ist gleich oder verschieden bei jedem Auftreten eine Gruppe der folgenden Formel (2), 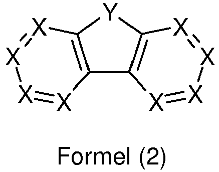 wobei die Struktur der Formel (2) an beliebigen Positionen mit Ar² und Ar³ bzw. mit N bzw. mit weiteren Gruppen Ar¹ für n > 1 verknüpft sein kann;
Y ist N, wenn die Gruppe der Formel (2) über dieses N mit Ar² bzw. Ar³ bzw. N bzw. Ar¹ für n > 1 verknüpft ist, oder ist gleich oder verschieden bei jedem Auftreten NR, O, S, CR=CR, CR₂-CR₂ oder eine Gruppe der folgenden Formel (3), 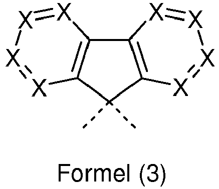 wobei die gestrichelten Bindungen die Verknüpfung der Gruppe kennzeichnen;
oder Y kann weiterhin auch für CR₂ stehen, wenn zwei benachbarten Gruppen X zusammen für eine Gruppe der Formel (4), (5) oder (6) stehen;
X ist C, wenn die Gruppe der Formel (2) über dieses X mit Ar² bzw. Ar³ bzw. N bzw. Ar¹ für n > 1 verknüpft ist, oder ist gleich oder verschieden bei jedem Auftreten CR oder N; oder zwei benachbarte Gruppen X stehen zusammen für eine Gruppe der folgenden Formel (4), (5) oder (6), 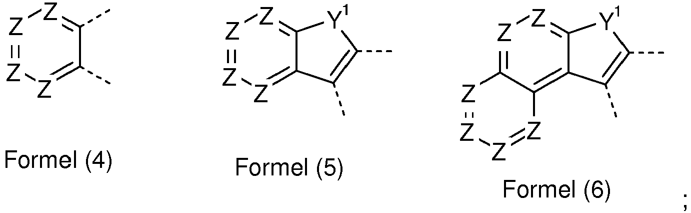 wobei die gestrichelten Bindungen die Verknüpfung der Gruppe kennzeichnen;
Z ist C, wenn die Gruppe der Formel (4) bzw. Formel (5) bzw. Formel (6) über dieses X mit Ar² bzw. Ar³ bzw. N bzw. Ar¹ für n > 1 verknüpft ist, oder ist bei jedem Auftreten gleich oder verschieden CR oder N;
Y¹ ist bei jedem Auftreten gleich oder verschieden CR₂, NR, O oder S;
Ar², Ar³ ist bei jedem Auftreten gleich oder verschieden eine Aryl- oder Heteroarylgruppe mit 5 bis 18 aromatischen Ringatomen, welche mit einem oder mehreren Resten R substituiert sein kann;
Ar⁴ bis Ar⁷ ist bei jedem Auftreten gleich oder verschieden ein aromatisches oder heteroaromatisches Ringsystem mit 5 bis 24 aromatischen Ringatomen, welches durch einen oder mehrere Reste R substituiert sein kann;
R ist bei jedem Auftreten gleich oder verschieden H, D, F, Cl, Br, I, CN, NO₂, Si(R¹)3, B(OR¹)₂, C(=O)R¹, P(=O)(R¹)₂, S(=O)R¹, S(=O)₂R¹, OSO₂R¹, eine geradkettige Alkyl-, Alkoxy- oder Thioalkoxygruppe mit 1 bis 40 C-Atomen oder eine Alkenyl- oder Alkinylgruppe mit 2 bis 40 C-Atomen oder eine verzweigte oder cyclische Alkyl-, Alkoxy- oder Thioalkoxygruppe mit 3 bis 40 C-Atomen, die jeweils mit einem oder mehreren Resten R¹ substituiert sein kann, wobei eine oder mehrere nicht benachbarte CH₂-Gruppen durch R¹C=CR¹, C=C, Si(R¹)₂, C=O, C=S, C=NR¹, P(=O)(R¹), SO, SO₂, NR¹, O, S oder CONR¹ ersetzt sein können und wobei ein oder mehrere H-Atome durch D, F, Cl, Br, I oder CN ersetzt sein können, oder ein aromatisches oder heteroaromatisches Ringsystem mit 5 bis 60 aromatischen Ringatomen, das jeweils durch einen oder mehrere Reste R¹ substituiert sein kann, oder eine Aryloxy- oder Heteroaryloxygruppe mit 5 bis 60 aromatischen Ringatomen, die durch einen oder mehrere Reste R¹ substituiert sein kann, oder eine Aralkyl- oder Heteroaralkylgruppe mit 5 bis 60 aromatischen Ringatomen, die durch einen oder mehrere Reste R¹ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R¹ substituiert sein kann; dabei können zwei oder mehrere Reste R auch miteinander ein mono- oder polycyclisches, aliphatisches, aromatisches und/oder benzoannelliertes Ringsystem bilden;
R¹ ist bei jedem Auftreten gleich oder verschieden H, D, F oder ein aliphatischer, aromatischer und/oder heteroaromatischer Kohlenwasserstoffrest mit 1 bis 20 C-Atomen, in dem auch ein oder mehrere H-Atome durch F ersetzt sein können; dabei können zwei oder mehrere Substituenten R¹ auch miteinander ein mono- oder polycyclisches, aliphatisches oder aromatisches Ringsystem bilden;
n ist 2, 3 oder 4;
m ist 1;
q, r ist gleich oder verschieden bei jedem Auftreten 0, 1, 2 oder 3;
**dadurch gekennzeichnet, dass** mindestens zwei der Gruppen Ar⁴ bis Ar⁷ jeweils durch eine Gruppe der folgenden Formel (7) substituiert sind:
---L-(Ar⁸)ₚ-Q Formel (7)
worin gilt:
L ist gleich oder verschieden bei jedem Auftreten eine Abstandsgruppe oder eine direkte Bindung;
Ar⁸ ist gleich oder verschieden bei jedem Auftreten ein aromatisches oder heteroaromatisches Ringsystem mit 5 bis 24 aromatischen Ringatomen, welches durch einen oder mehrere Reste R substituiert sein kann;
Q ist gleich oder verschieden bei jedem Auftreten eine vernetzbare Gruppe, ausgewählt aus endständigen oder cyclischen Alkenylgruppen, endständigen Alkinylgruppen, Arylvinylgruppen, Acrylsäurederivaten, Alkenyloxy- bzw. Perfluoralkenyloxyderivate, Gruppen, die eine Ringöffnungspolymerisation eingehen, insbesondere Oxetan- und Oxiranderivaten, oder Silanen;
p ist gleich oder verschieden bei jedem Auftreten 0 oder 1.

2. Verbindung nach Anspruch 1, **dadurch gekennzeichnet, dass** die Gruppe Ar¹ gleich oder verschieden bei jedem Auftreten ausgewählt ist aus den Gruppen der Formeln (8) bis (27), 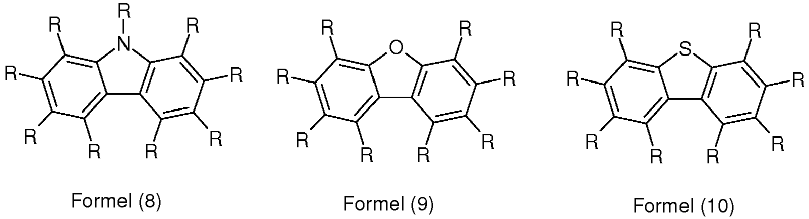 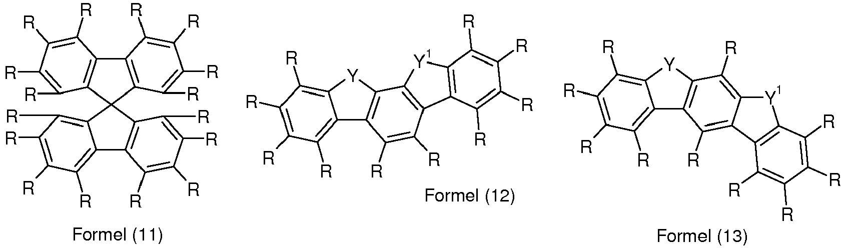 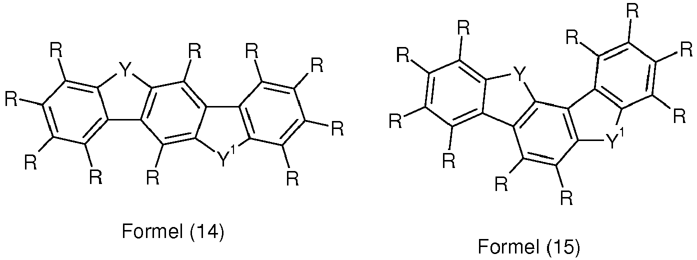 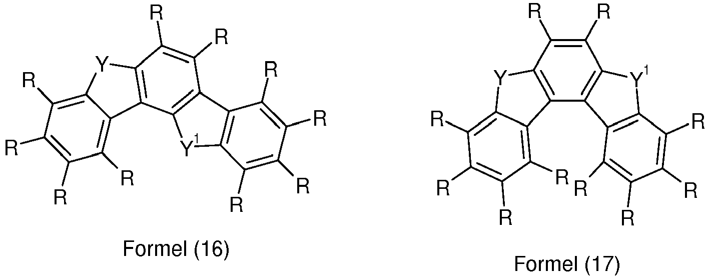 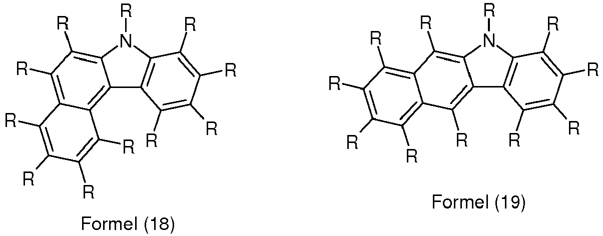 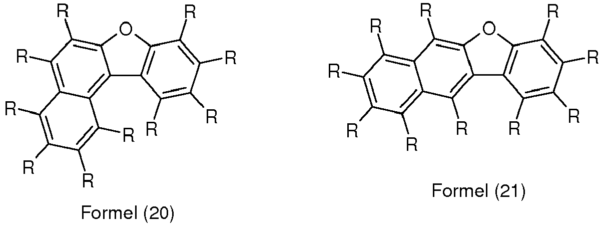 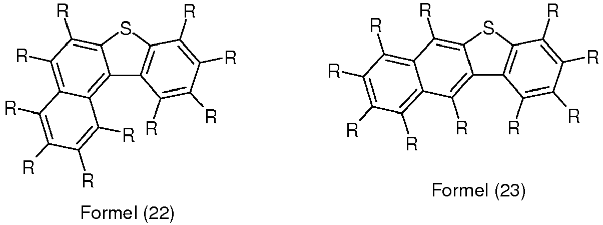 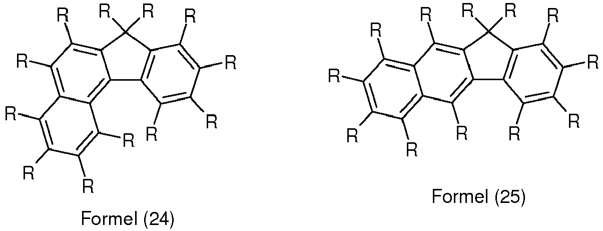 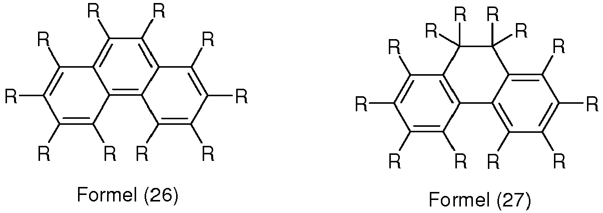 wobei Y, Y¹ und R die in Anspruch 1 genannten Bedeutungen aufweisen und kein Rest R an den Positionen vorhanden ist, an denen die Struktur an Ar² bzw. Ar³ bzw. an den Stickstoff bzw. an eine weitere Gruppe Ar¹ für n > 1 gebunden ist; dabei können die Gruppen jeweils über beliebige Positionen binden.

3. Verbindung nach Anspruch 1 oder 2, **dadurch gekennzeichnet, dass** die Gruppe Ar¹ gleich oder verschieden bei jedem Auftreten ausgewählt ist aus den Strukturen der Formeln (8a) bis (27b), 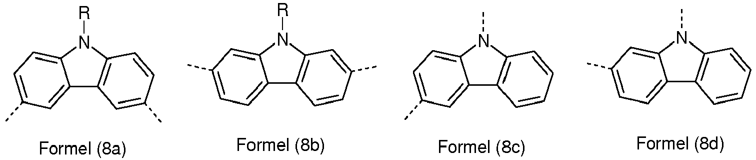 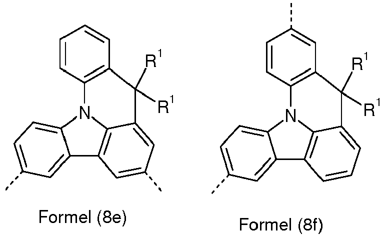 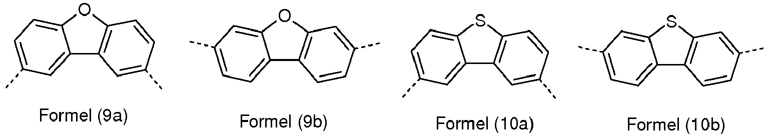 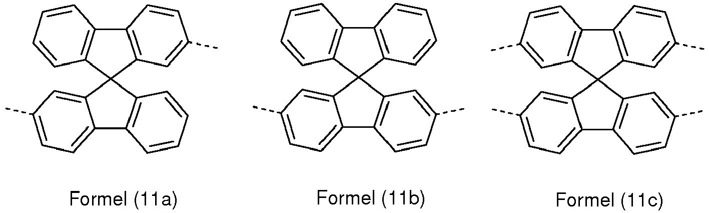 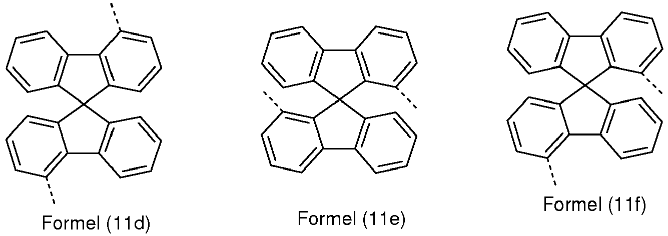 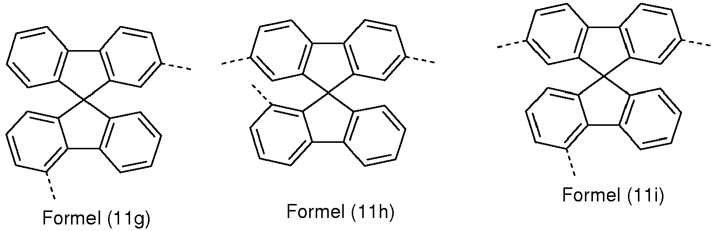 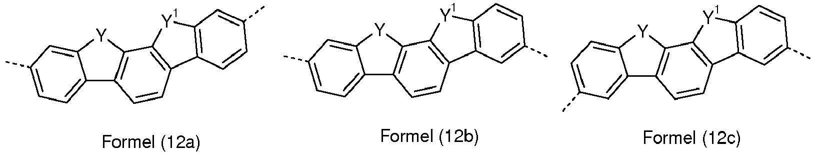 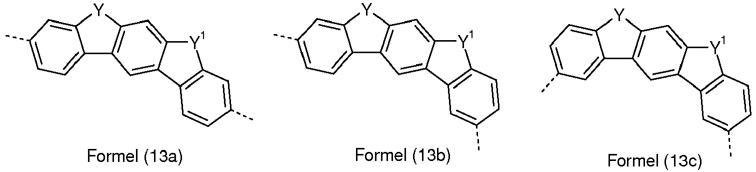 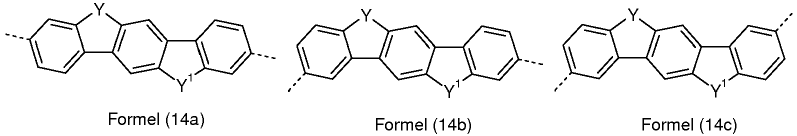 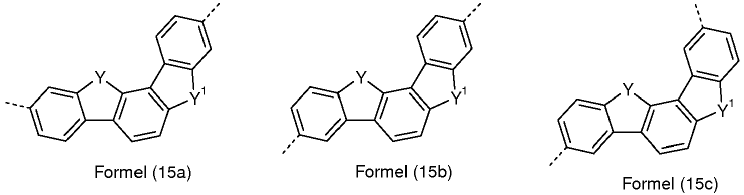 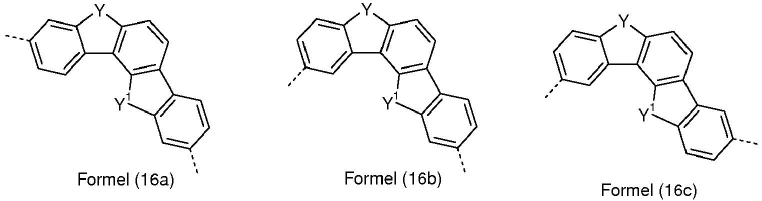 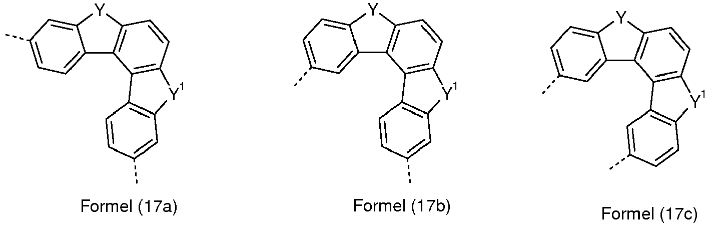 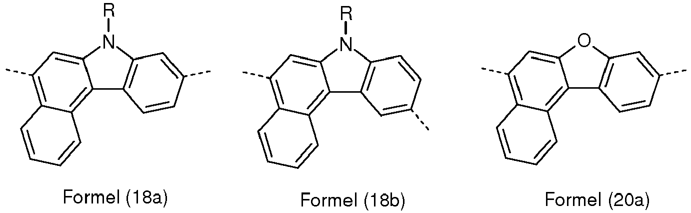 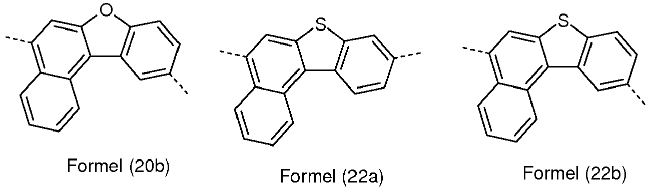 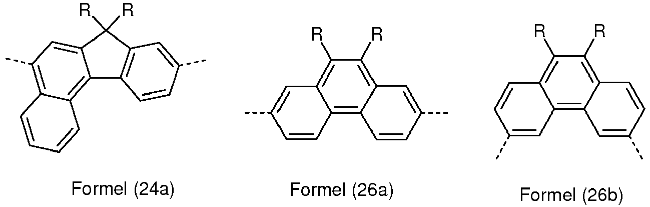 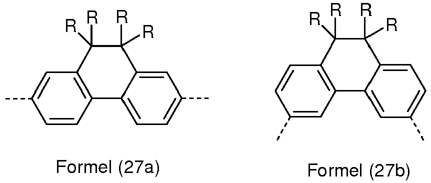 wobei Y, Y¹ und R die in Anspruch 1 genannten Bedeutungen aufweisen und die gestrichelte Bindung die Position andeutet, an denen die Struktur an Ar² bzw. Ar³ bzw. an den Stickstoff bzw. an Ar¹ für n > 1 gebunden ist.

4. Verbindung nach einem oder mehreren der Ansprüche 1 bis 3, **dadurch gekennzeichnet, dass** Ar² und Ar³ gleich oder verschieden bei jedem Auftreten ausgewählt ist aus 1,2-Phenylen, 1,3-Phenylen oder 1,4-Phenylen, welches jeweils unsubstituiert oder durch einen oder mehrere Reste R substituiert sein kann.

5. Verbindung nach einem oder mehreren der Ansprüche 1 bis 4, **dadurch gekennzeichnet, dass** Ar⁴ bis Ar⁷ gleich oder verschieden bei jedem Auftreten ausgewählt ist aus der Gruppe bestehend aus Phenyl, ortho-, meta- oder para-Biphenyl, ortho-, meta- oder para-Terphenyl, lineares oder verzweigtes Quaterphenyl, Fluorenyl, Spirobifluorenyl oder Carbazolyl, welches jeweils durch einen oder mehrere Reste R substituiert sein kann.

6. Verbindung nach einem oder mehreren der Ansprüche 1 bis 5, **dadurch gekennzeichnet, dass** die Verbindung zwischen 2 und 8 Gruppen der Formel (7), vorzugsweise 2, 3 oder 4 Gruppen der Formel (7) enthält.

7. Verbindung nach einem oder mehreren der Ansprüche 1 bis 6, **dadurch gekennzeichnet, dass** L eine Einfachbindung ist oder dass L eine lineare oder verzweigte Alkylen-Gruppe mit 1 bis 20 C-Atomen ist, vorzugsweise mit 1 bis 12 C-Atomen, in der eine oder mehrere nicht benachbarte CH₂-Gruppen durch -O-, -S-, -NH-, -N(CH₃)-, -N-CO-, -N-CO-O-, -N-CO-N, -CO-, -O-CO-, -S-CO-, -O-COO-, -CO-S-, - CO-O-, -CH(Halogen)-, -CH(CN)-, -CH=CH- oder -C≡C- ersetzt sein können, oder eine cyclische Alkylgruppe, vorzugsweise Cyclohexan oder ein Cyclohexanderivat mit 1,4- oder 1,3-Verknüpfung.

8. Verbindung nach einem oder mehreren der Ansprüche 1 bis 7, **dadurch gekennzeichnet, dass** Ar⁸ ausgewählt ist aus 1,2-Phenylen, 1,3-Phenylen oder 1,4-Phenylen, welches jeweils unsubstituiert oder durch einen oder mehrere Reste R substituiert sein kann.

9. Verbindung bzw. Schicht, **dadurch gekennzeichnet, dass** sie durch Vernetzung der Gruppen Q einer Verbindung nach einem oder mehreren der Ansprüche 1 bis 8 erhältlich ist, wobei die Vernetzung gegebenenfalls in einer Schicht durchgeführt wird.

10. Verfahren zur Herstellung einer vernetzten Schicht, **dadurch gekennzeichnet, dass** die Verbindung nach einem oder mehreren der Ansprüche 1 bis 8 aus Lösung zu einer Schicht aufgebracht wird und diese Schicht vernetzt wird.

11. Verwendung einer Verbindung nach einem oder mehreren der Ansprüche 1 bis 8 bzw. einer Verbindung oder Schicht nach Anspruch 9 in einer elektronischen Vorrichtung.

12. Elektronische Vorrichtung, insbesondere organische Elektrolumineszenzvorrichtung, organische integrierte Schaltung, organischer Feld-Effekt-Transistor, organischer Dünnfilmtransistor, organischer lichtemittierender Transistor, organische Solarzelle, organischer optischer Detektor, organischer Fotorezeptor, organisches Feld-Quench-Device, lichtemittierende elektrochemische Zelle oder organische Laserdiode, enthaltend eine oder mehrere Verbindungen nach einem oder mehreren der Ansprüche 1 bis 8 bzw. eine Verbindung oder Schicht nach Anspruch 9.

13. Elektronische Vorrichtung nach Anspruch 12, welche eine organische Elektrolumineszenzvorrichtung ist, umfassend den folgenden Aufbau: Anode / optional Schicht aus einem leitfähigen Polymer / eine oder mehrere Schichten nach Anspruch 9 / Emissionsschicht und Kathode.

14. Organische Elektrolumineszenzvorrichtung nach Anspruch 12 oder 13, **dadurch gekennzeichnet, dass** die Verbindungen nach einem oder mehreren der Ansprüche 1 bis 8 bzw. die Verbindungen nach Anspruch 9 in einer Lochtransportschicht bzw. in einer Lochinjektionsschicht verwendet werden, wobei diese Schicht auch dotiert sein kann, insbesondere mit einer Elektronenakzeptorverbindung.

15. Formulierung, enthaltend mindestens eine Verbindung nach einem oder mehreren der Ansprüche 1 bis 8 und ein oder mehrere Lösungsmittel.

## Claims

1. Compound of the formula (1), 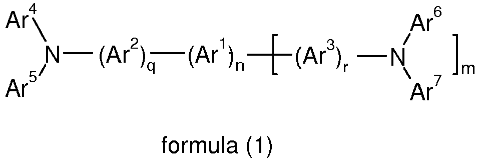 where the following applies to the symbols and indices used:
Ar¹ is, identically or differently on each occurrence, a group of the following formula (2), 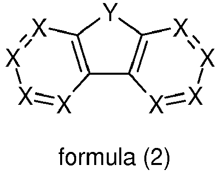 where the structure of the formula (2) can be linked at any desired positions to Ar² and Ar³ or to N or to further groups Ar¹ for n > 1;
Y is N if the group of the formula (2) is linked via this N to Ar² or Ar³ or N or Ar¹ for n > 1 or is, identically or differently on each occurrence, NR, O, S, CR=CR, CR₂-CR₂ or a group of the following formula (3), 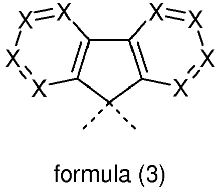 where the dashed bonds denote the linking of the group;
or Y may furthermore also stand for CR₂ if two adjacent groups X together stand for a group of the formula (4), (5) or (6);
X is C if the group of the formula (2) is linked via this X to Ar² or Ar³ or N or Ar¹ for n > 1 or is, identically or differently on each occurrence, CR or N; or two adjacent groups X together stand for a group of the following formula (4), (5) or (6), 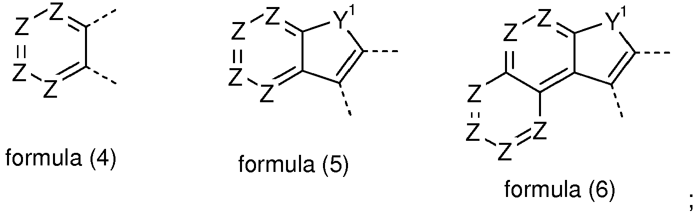 where the dashed bonds denote the linking of the group;
Z is C if the group of the formula (4) or formula (5) or formula (6) is linked via this X to Ar² or Ar³ or N or Ar¹ for n > 1 or is on each occurrence, identically or differently, CR or N;
Y¹ is on each occurrence, identically or differently, CR₂, NR, O or S;
Ar², Ar³ are on each occurrence, identically or differently, an aryl or heteroaryl group having 5 to 18 aromatic ring atoms, which may be substituted by one or more radicals R;
Ar⁴ to Ar⁷ are on each occurrence, identically or differently, an aromatic or heteroaromatic ring system having 5 to 24 aromatic ring atoms, which may be substituted by one or more radicals R;
R is on each occurrence, identically or differently, H, D, F, Cl, Br, I, CN, NO₂, Si(R¹)3, B(OR¹)₂, C(=O)R¹, P(=O)(R¹)₂, S(=O)R¹, S(=O)₂R¹, OSO₂R¹, a straight-chain alkyl, alkoxy or thioalkoxy group having 1 to 40 C atoms or an alkenyl or alkynyl group having 2 to 40 C atoms or a branched or cyclic alkyl, alkoxy or thioalkoxy group having 3 to 40 C atoms, which may in each case be substituted by one or more radicals R¹, where one or more non-adjacent CH₂ groups may be replaced by R¹C=CR¹, C=C, Si(R¹)₂, C=O, C=S, C=NR¹, P(=O)(R¹), SO, SO₂, NR¹, O, S or CONR¹ and where one or more H atoms may be replaced by D, F, Cl, Br, I or CN, or an aromatic or heteroaromatic ring system having 5 to 60 aromatic ring atoms, which may in each case be substituted by one or more radicals R¹, or an aryloxy or heteroaryloxy group having 5 to 60 aromatic ring atoms, which may be substituted by one or more radicals R¹, or an aralkyl or heteroaralkyl group having 5 to 60 aromatic ring atoms, which may be substituted by one or more radicals R¹, or a diarylamino group, diheteroarylamino group or arylheteroarylamino group having 10 to 40 aromatic ring atoms, which may be substituted by one or more radicals R¹; two or more radicals R may also form a mono- or polycyclic, aliphatic, aromatic and/or benzo-fused ring system with one another;
R¹ is on each occurrence, identically or differently, H, D, F or an aliphatic, aromatic and/or heteroaromatic hydrocarbon radical having 1 to 20 C atoms, in which, in addition, one or more H atoms may be replaced by F; two or more substituents R¹ may also form a mono- or polycyclic, aliphatic or aromatic ring system with one another;
n is 2, 3 or 4;
m is 1;
q, r are, identically or differently on each occurrence, 0, 1, 2 or 3;
**characterised in that** at least two of the groups Ar⁴ to Ar⁷ are in each case substituted by a group of the following formula (7):
---L-(Ar⁸)ₚ-Q formula (7)
in which:
L is, identically or differently on each occurrence, a spacer group or a direct bond;
Ar⁸ is, identically or differently on each occurrence, an aromatic or heteroaromatic ring system having 5 to 24 aromatic ring atoms, which may be substituted by one or more radicals R;
Q is, identically or differently on each occurrence, a crosslinkable group selected from terminal or cyclic alkenyl groups, terminal alkynyl groups, arylvinyl groups, acrylic acid derivatives, alkenyloxy or perfluoroalkenyloxy derivatives, groups which undergo a ring-opening polymerisation, in particular oxetane and oxirane derivatives, or silanes;
p is, identically or differently on each occurrence, 0 or 1.

2. Compound according to Claim 1, **characterised in that** the group Ar¹ is selected, identically or differently on each occurrence, from the groups of the formulae (8) to (27), 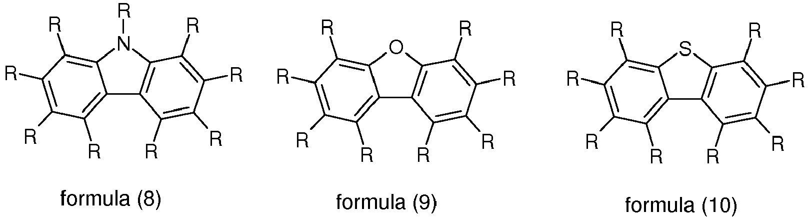 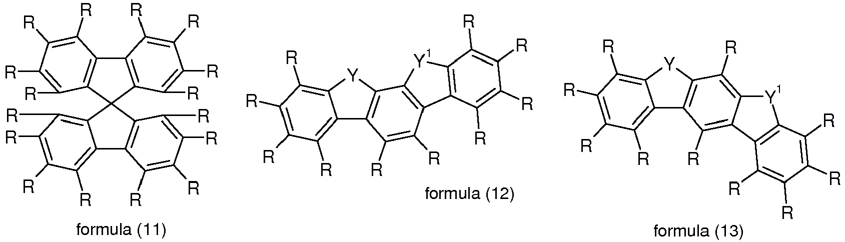 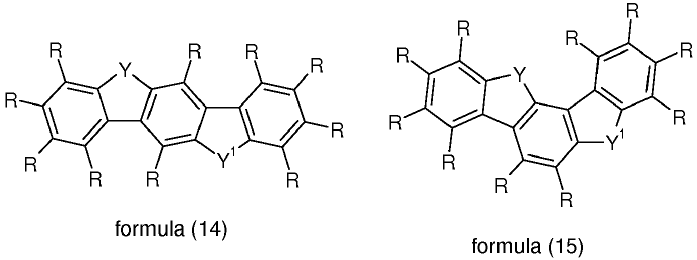 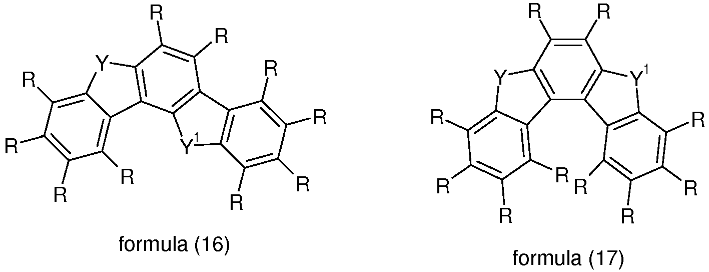 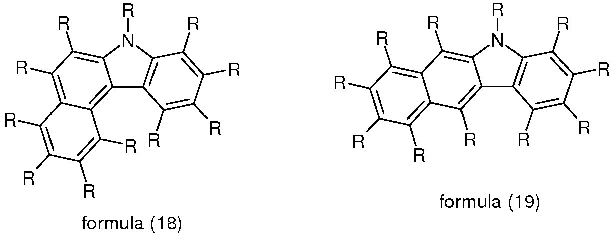 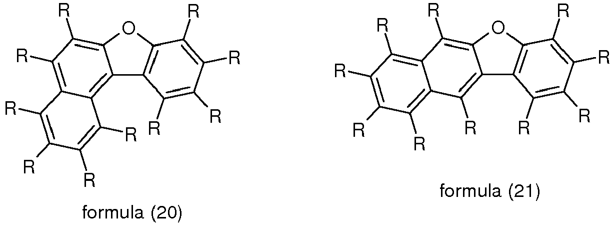 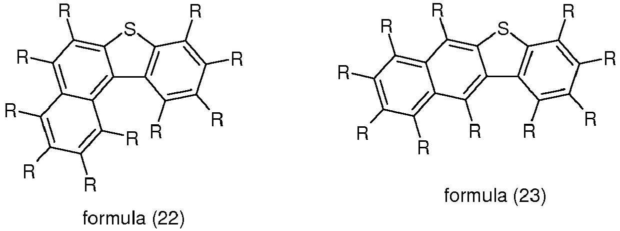 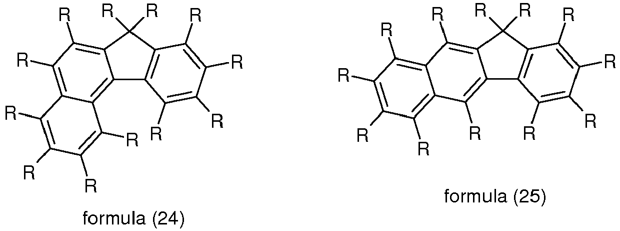 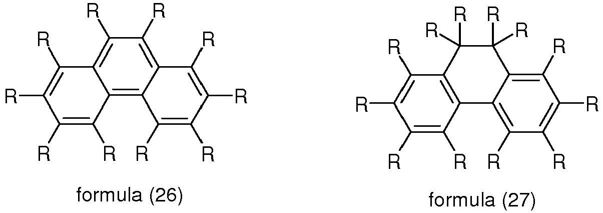 where Y, Y¹ and R have the meanings given in Claim 1 and no radical R is present at the positions at which the structure is bonded to Ar² or Ar³ or to the nitrogen or to a further group Ar¹ for n > 1; the groups can in each case be bonded via any desired positions.

3. Compound according to Claim 1 or 2, **characterised in that** the group Ar¹ is selected, identically or differently on each occurrence, from the structures of the formulae (8a) to (27b), 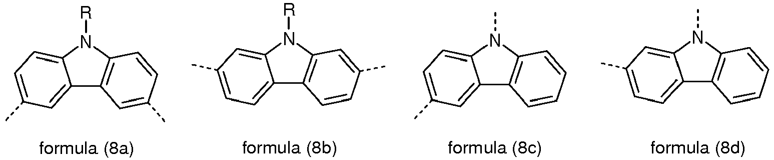 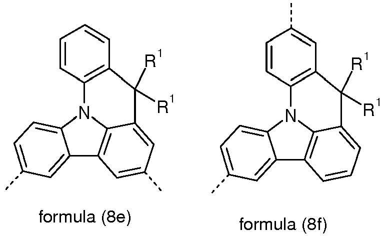 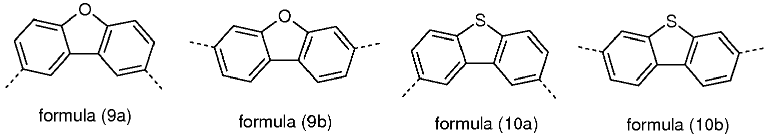 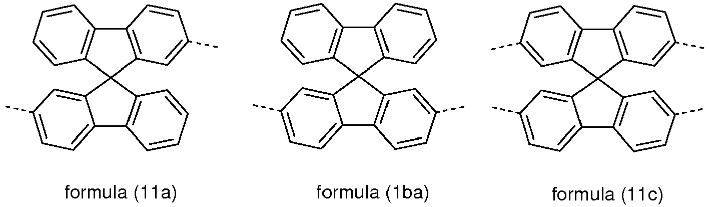 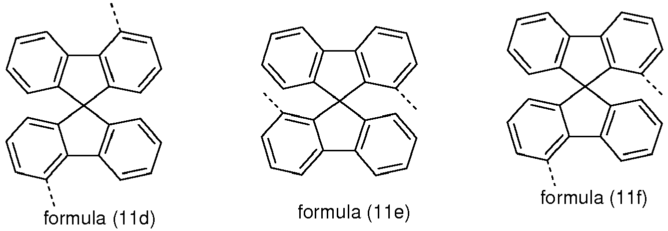 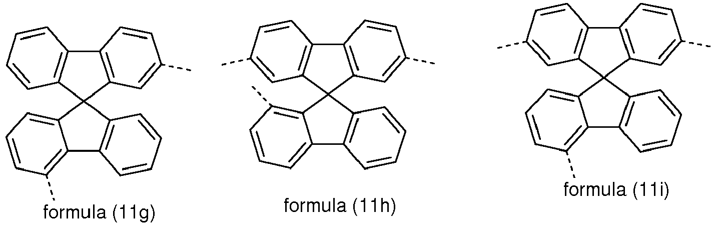 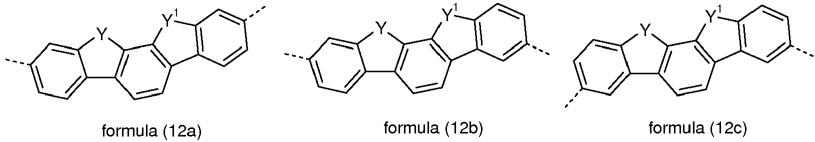 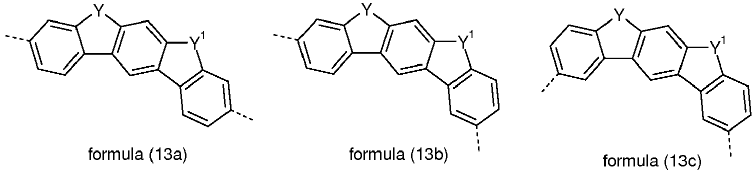 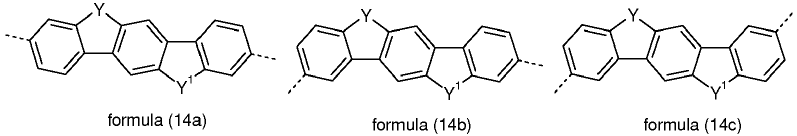 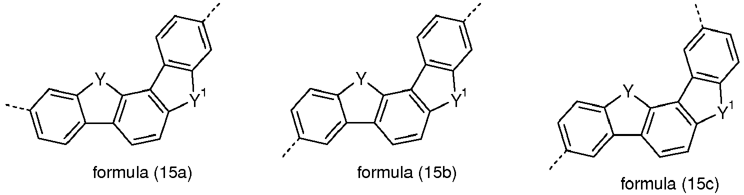 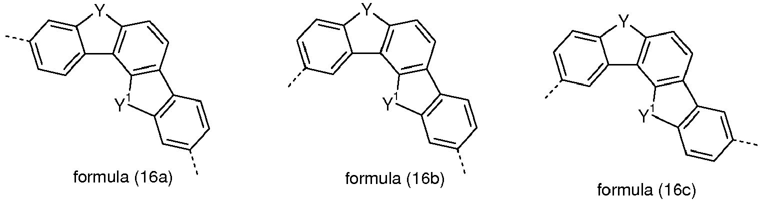 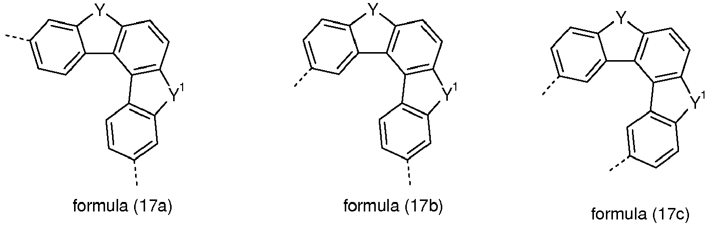 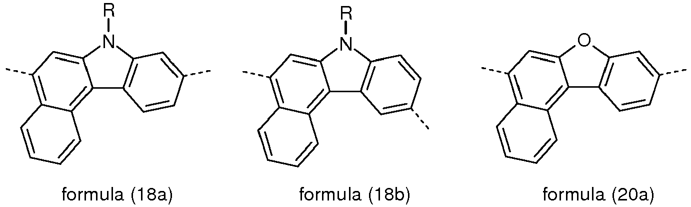 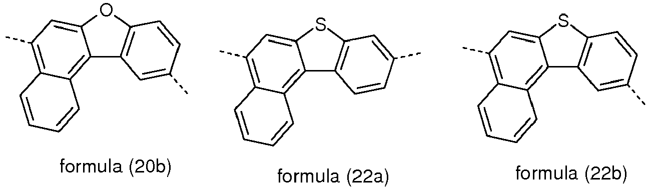 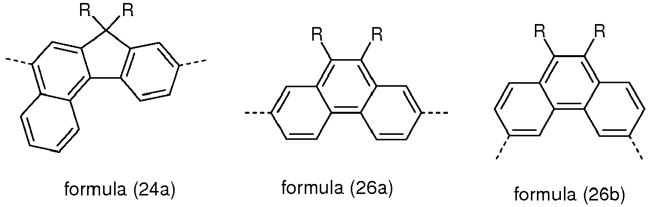 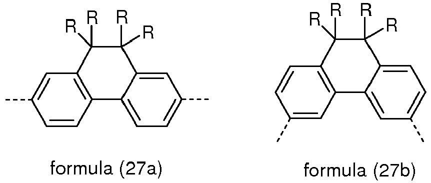 where Y, Y¹ and R have the meanings given in Claim 1 and the dashed bond indicates the position at which the structure is bonded to Ar² or Ar³ or to the nitrogen or to Ar¹ for n > 1.

4. Compound according to one or more of Claims 1 to 3, **characterised in that** Ar² and Ar³ are selected, identically or differently on each occurrence, from 1,2-phenylene, 1,3-phenylene or 1,4-phenylene, which may in each case be unsubstituted or substituted by one or more radicals R.

5. Compound according to one or more of Claims 1 to 4, **characterised in that** Ar⁴ to Ar⁷ are selected, identically or differently on each occurrence, from the group consisting of phenyl, ortho-, meta- or para-biphenyl, ortho-, meta- or para-terphenyl, linear or branched quaterphenyl, fluorenyl, spirobifluorenyl or carbazolyl, which may in each case be substituted by one or more radicals R.

6. Compound according to one or more of Claims 1 to 5, **characterised in that** the compound contains between 2 and 8 groups of the formula (7), preferably 2, 3 or 4 groups of the formula (7).

7. Compound according to one or more of Claims 1 to 6, **characterised in that** L is a single bond or **in that** L is a linear or branched alkylene group having 1 to 20 C atoms, preferably having 1 to 12 C atoms, in which one or more non-adjacent CH₂ groups may be replaced by -O-, -S-, -NH-, -N(CH₃)-, -N-CO-, -N-CO-O-, -N-CO-N, -CO-, -O-CO-, -S-CO-, -O-COO-, -CO-S-, -CO-O-, -CH(halogen)-, -CH(CN)-, -CH=CH- or -C≡C-, or a cyclic alkyl group, preferably cyclohexane or a cyclohexane derivative having 1,4- or 1,3-linking.

8. Compound according to one or more of Claims 1 to 7, **characterised in that** Ar⁸ is selected from 1,2-phenylene, 1,3-phenylene or 1,4-phenylene, which may in each case be unsubstituted or substituted by one or more radicals R.

9. Compound or layer, **characterised in that** it is obtainable by crosslinking the groups Q of a compound according to one or more of Claims 1 to 8, where the crosslinking is optionally carried out in a layer.

10. Process for the production of a crosslinked layer, **characterised in that** the compound according to one or more of Claims 1 to 8 is applied from solution to give a layer, and this layer is crosslinked.

11. Use of a compound according to one or more of Claims 1 to 8 or a compound or layer according to Claim 9 in an electronic device.

12. Electronic device, in particular organic electroluminescent device, organic integrated circuit, organic field-effect transistor, organic thin-film transistor, organic light-emitting transistor, organic solar cell, organic optical detector, organic photoreceptor, organic field-quench device, light-emitting electrochemical cell or organic laser diode, comprising one or more compounds according to one or more of Claims 1 to 8 or a compound or layer according to Claim 9.

13. Electronic device according to Claim 12, which is an organic electroluminescent device, comprising the following structure: anode / optionally layer comprising a conductive polymer / one or more layers according to Claim 9 / emission layer and cathode.

14. Organic electroluminescent device according to Claim 12 or 13, **characterised in that** the compounds according to one or more of Claims 1 to 8 or the compounds according to Claim 9 are used in a hole-transport layer or in a hole-injection layer, where this layer may also be doped, in particular by an electron-acceptor compound.

15. Formulation comprising at least one compound according to one or more of Claims 1 to 8 and one or more solvents.

## Revendications

1. Composé de la formule (1) : 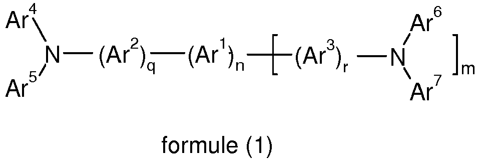 dans laquelle ce qui suit s'applique aux symboles et aux indices qui sont utilisés :
Ar¹ est, de manière identique ou différente pour chaque occurrence, un groupe de la formule (2) qui suit : 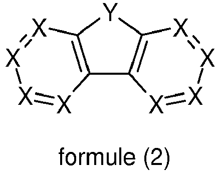 dans laquelle la structure de la formule (2) peut être liée au niveau de n'importe quelles positions souhaitées à Ar² et à Ar³ ou à N ou à d'autres groupes Ar¹ pour n > 1 ;
Y est N si le groupe de la formule (2) est lié via ce N à Ar² ou à Ar³ ou à N ou à Ar¹ pour n > 1 ou est, de manière identique ou différente pour chaque occurrence, NR, O, S, CR=CR, CR₂-CR₂ ou un groupe de la formule (3) qui suit : 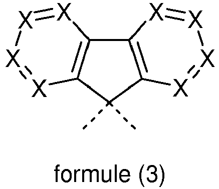 dans laquelle les liens en pointillés représentent la liaison du groupe ;
ou Y peut en outre représenter également CR₂ si deux groupes X adjacents représentent en association un groupe de la formule (4), (5) ou (6) ;
X est C si le groupe de la formule (2) est lié via ce X à Ar² ou à Ar³ ou à N ou à Ar¹ pour n > 1 ou est, de manière identique ou différente pour chaque occurrence, CR ou N ; ou deux groupes X adjacents représentent en association un groupe de la formule (4), (5) ou (6) qui suit : 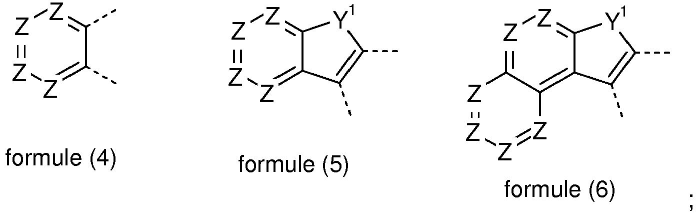 dans lesquelles les liens en pointillés représentent la liaison du groupe ;
Z est C si le groupe de la formule (4) ou de la formule (5) ou de la formule (6) est lié via ce X à Ar² ou à Ar³ ou à N ou à Ar¹ pour n > 1 ou est pour chaque occurrence, de manière identique ou différente, CR ou N ;
Y¹ est pour chaque occurrence, de manière identique ou différente, CR₂, NR, O ou S ;
Ar², Ar³ sont pour chaque occurrence, de manière identique ou différente, un groupe aryle ou hétéroaryle qui comporte de 5 à 18 atomes de cycle aromatique, lequel peut être substitué par un radical ou par plusieurs radicaux R ;
Ar⁴ à Ar⁷ sont pour chaque occurrence, de manière identique ou différente, un système de cycle aromatique ou hétéroaromatique qui comporte de 5 à 24 atomes de cycle aromatique, lequel peut être substitué par un radical ou par plusieurs radicaux R ;
R est pour chaque occurrence, de manière identique ou différente, H, D, F, Cl, Br, I, CN, NO₂, Si(R¹)3, B(OR¹)₂, C(=O)R¹, P(=O)(R¹)₂, S(=O)R¹, S(=O)₂R¹, OSO₂R¹, un groupe alkyle, alcoxy ou thioalcoxy en chaîne droite qui comporte de 1 à 40 atome(s) de C ou un groupe alkényle ou alkynyle qui comporte de 2 à 40 atomes de C ou un groupe alkyle, alcoxy ou thioalcoxy ramifié ou cyclique qui comporte de 3 à 40 atomes de C, lequel peut dans chaque cas être substitué par un radical ou par plusieurs radicaux R¹, où un ou plusieurs groupe(s) CH₂ non adjacents peut/peuvent être remplacé(s) par R¹C=CR¹, C=C, Si(R¹)₂, C=O, C=S, C=NR¹, P(=O)(R¹), SO, SO₂, NR¹, O, S ou CONR¹ et où un ou plusieurs atome(s) de H peut/peuvent être remplacé(s) par D, F, Cl, Br, I ou CN, ou un système de cycle aromatique ou hétéroaromatique qui comporte de 5 à 60 atomes de cycle aromatique, lequel peut dans chaque cas être substitué par un radical ou par plusieurs radicaux R¹, ou un groupe aryloxy ou hétéroaryloxy qui comporte de 5 à 60 atomes de cycle aromatique, lequel peut être substitué par un radical ou par plusieurs radicaux R¹, ou un groupe aralkyle ou hétéroaralkyle qui comporte de 5 à 60 atomes de cycle aromatique, lequel peut être substitué par un radical ou par plusieurs radicaux R¹, ou un groupe diarylamino, un groupe dihétéroaryl-amino ou un groupe arylhétéroarylamino qui comporte de 10 à 40 atomes de cycle aromatique, lequel peut être substitué par un radical ou par plusieurs radicaux R¹ ; deux radicaux R ou plus peuvent également former un système de cycle aliphatique, aromatique mono- ou polycyclique et/ou benzo-fusionné l'un avec l'autre ou les uns avec les autres ;
R¹ est pour chaque occurrence, de manière identique ou différente, H, D, F ou un radical hydrocarbone aliphatique, aromatique et/ou hétéroaromatique qui comporte de 1 à 20 atome(s) de C, où, en outre, un ou plusieurs atome(s) de H peut/peuvent être remplacé(s) par F ; deux substituants R¹ ou plus peuvent également former un système de cycle aliphatique ou aromatique mono- ou polycyclique l'un avec l'autre ou les uns avec les autres ;
n est 2, 3 ou 4 ;
m est 1 ;
q, r sont, de manière identique ou différente pour chaque occurrence, 0, 1, 2 ou 3 ;
**caractérisé en ce qu'**au moins deux des groupes Ar⁴ à Ar⁷ sont dans chaque cas substitués par un groupe de la formule (7) qui suit :
---L-(Ar⁸)ₚ-Q formule (7)
dans laquelle :
L est, de manière identique ou différente pour chaque occurrence, un groupe d'espaceur ou une liaison directe ;
Ar⁸ est, de manière identique ou différente pour chaque occurrence, un système de cycle aromatique ou hétéroaromatique qui comporte de 5 à 24 atomes de cycle aromatique, lequel peut être substitué par un radical ou par plusieurs radicaux R ;
Q est, de manière identique ou différente pour chaque occurrence, un groupe réticulable qui est sélectionné parmi des groupes alkényle terminaux ou cycliques, des groupes alkynyle terminaux, des groupes arylvinyle, des dérivés d'acide acrylique, des dérivés d'alkényloxy ou de perfluoroalkényloxy, des groupes qui subissent une polymérisation en cycle ouvert, en particulier des dérivés d'oxétane et d'oxirane, ou des silanes ;
p est, de manière identique ou différente pour chaque occurrence, 0 ou 1.

2. Composé selon la revendication 1, **caractérisé en ce que** le groupe Ar¹ est sélectionné, de manière identique ou différente pour chaque occurrence, parmi les groupes des formules (8) à (27) : 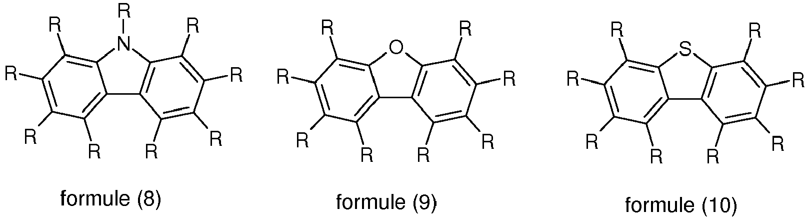 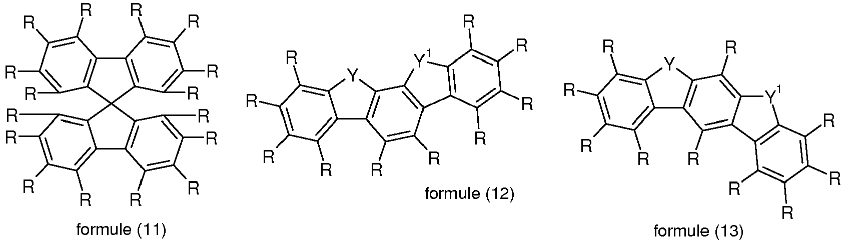 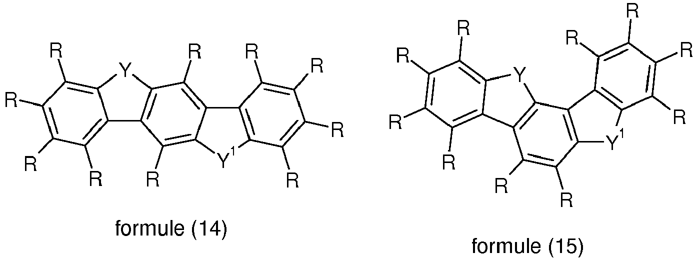 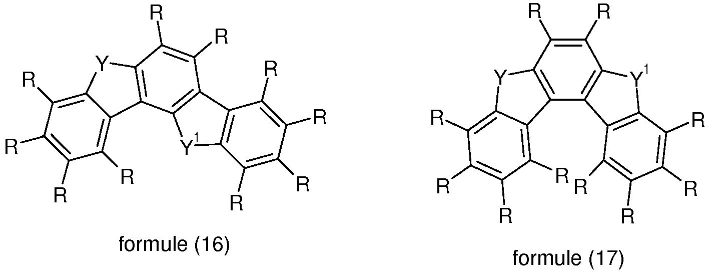 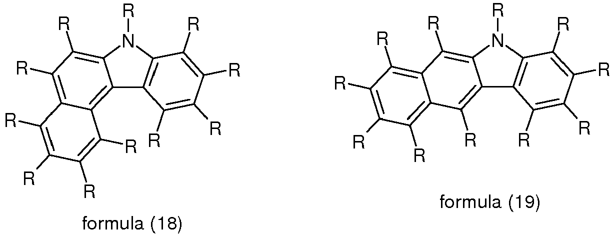 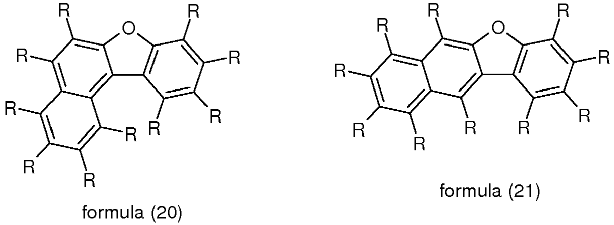 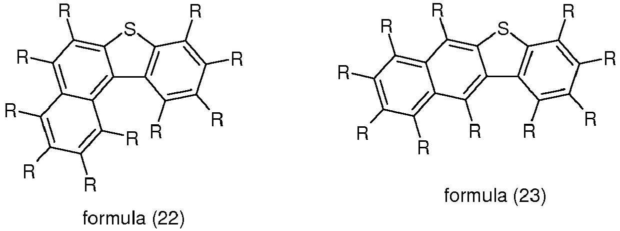 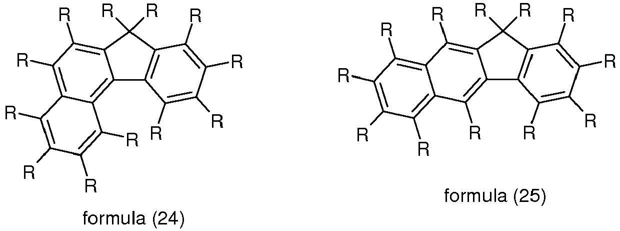 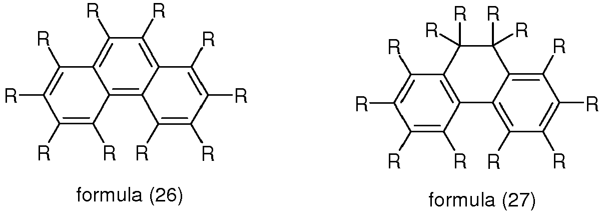 dans lesquelles Y, Y¹ et R présentent les significations qui ont été données selon la revendication 1 et aucun radical R n'est présent au niveau des positions au niveau desquelles la structure est liée à Ar² ou à Ar³ ou à l'azote ou à un autre groupe Ar¹ pour n > 1 ; les groupes peuvent dans chaque cas être liés via n'importe quelles positions souhaitées.

3. Composé selon la revendication 1 ou 2, **caractérisé en ce que** le groupe Ar¹ est sélectionné, de manière identique ou différente pour chaque occurrence, parmi les structures des formules (8a) à (27b) : 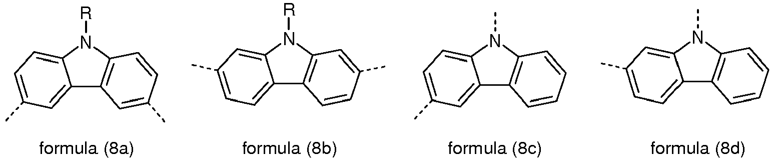 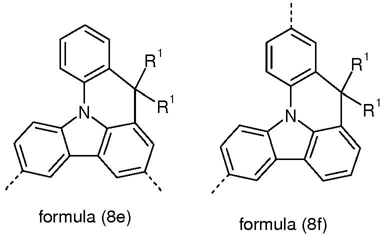 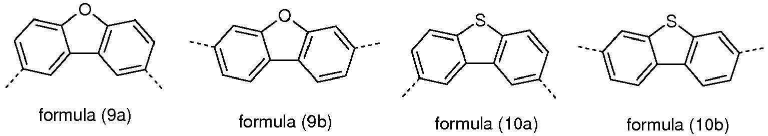 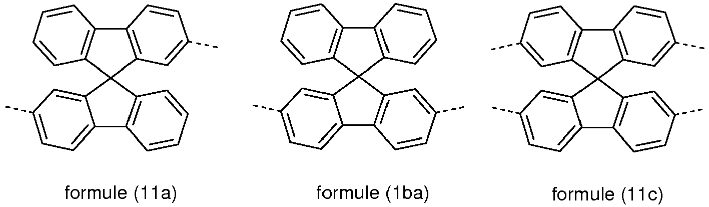 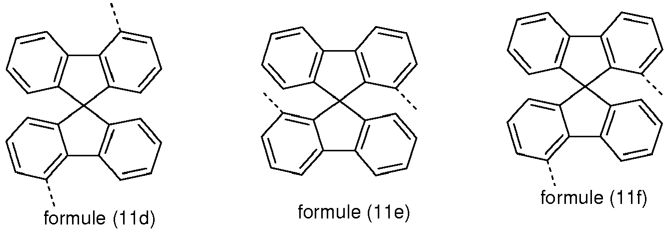 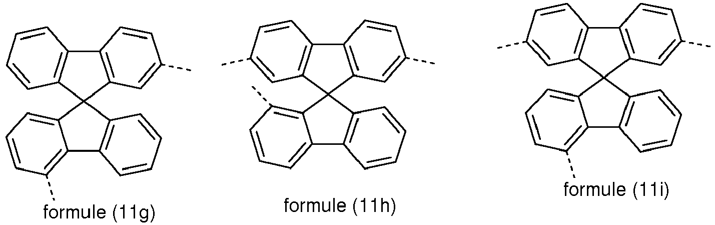 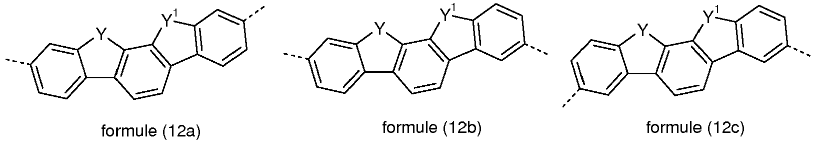 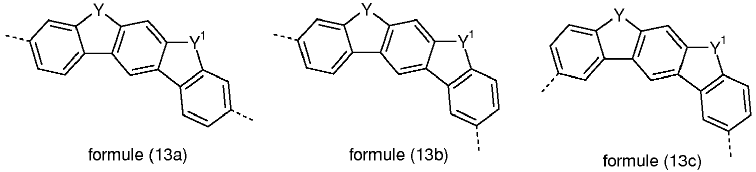 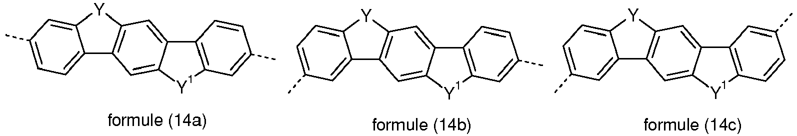 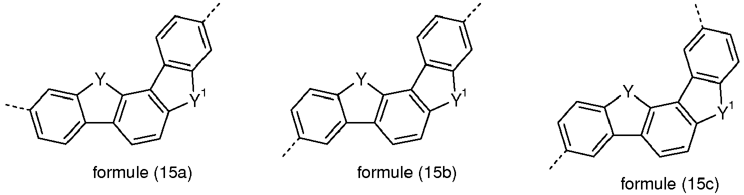 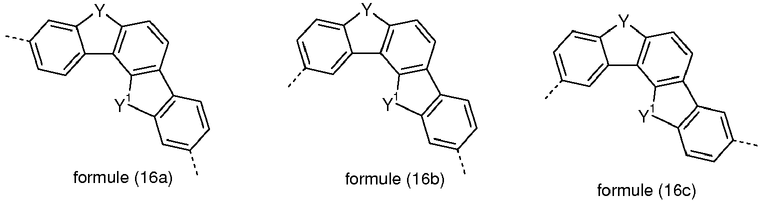 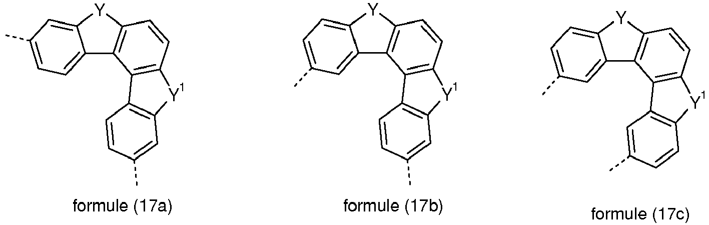 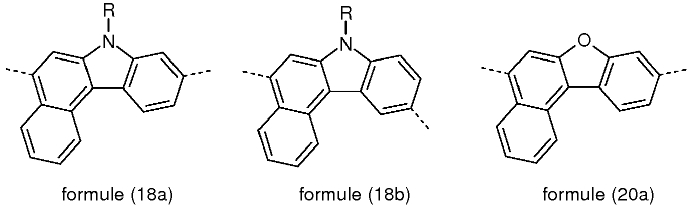 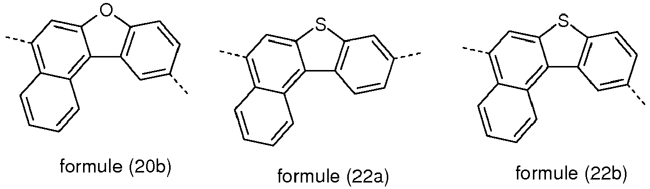 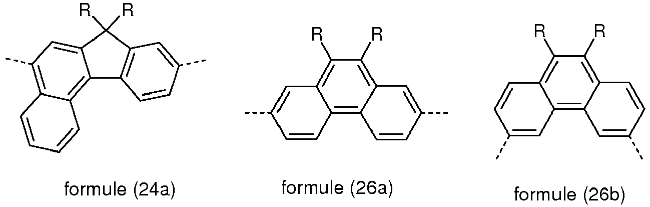 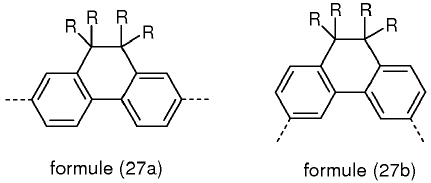 dans lesquelles Y, Y¹ et R présentent les significations qui ont été données selon la revendication 1 et le lien en pointillés indique la position au niveau de laquelle la structure est liée à Ar² ou à Ar³ ou à l'azote ou à Ar¹ pour n > 1.

4. Composé selon une ou plusieurs des revendications 1 à 3, **caractérisé en ce que** Ar² et Ar³ sont sélectionnés, de manière identique ou différente pour chaque occurrence, parmi 1,2-phénylène, 1,3-phénylène et 1,4-phénylène, lequel peut dans chaque cas être non substitué ou substitué par un radical ou par plusieurs radicaux R.

5. Composé selon une ou plusieurs des revendications 1 à 4, **caractérisé en ce que** Ar⁴ à Ar⁷ sont sélectionnés, de manière identique ou différente pour chaque occurrence, parmi le groupe qui est constitué par phényle, ortho-, méta- et para-biphényle, ortho-, méta- et para-terphényle, quaterphényle, fluorényle, spirobifluorényle et carbazolyle linéaire ou ramifié, lequel peut dans chaque cas être substitué par un radical ou par plusieurs radicaux R.

6. Composé selon une ou plusieurs des revendications 1 à 5, **caractérisé en ce que** le composé contient entre 2 et 8 groupes de la formule (7), de préférence 2, 3 ou 4 groupes de la formule (7).

7. Composé selon une ou plusieurs des revendications 1 à 6, **caractérisé en ce que** L est une liaison simple ou **en ce que** L est un groupe alkylène linéaire ou ramifié qui comporte de 1 à 20 atome(s) de C, de préférence qui comporte de 1 à 12 atome(s) de C, dans lequel un ou plusieurs groupe(s) CH₂ non adjacents peut/peuvent être remplacé(s) par -O-, -S-, -NH-, -N(CH₃)-, -N-CO-, -N-CO-O-, -N-CO-N, -CO-, -O-CO-, -S-CO-, -O-COO-, -CO-S-, -CO-O-, -CH(halogène)-, -CH(CN)-, -CH=CH- ou -C≡C-, ou un groupe alkyle cyclique, de préférence cyclohexane ou un dérivé de cyclohexane qui comporte une 1,4- ou 1,3-liaison.

8. Composé selon une ou plusieurs des revendications 1 à 7, **caractérisé en ce que** Ar⁸ est sélectionné parmi 1,2-phénylène, 1,3-phénylène et 1,4-phénylène, lequel peut dans chaque cas être non substitué ou substitué par un radical ou par plusieurs radicaux R.

9. Composé ou couche, caractérisé(e) en ce qu'il/elle peut être obtenu(e) en réticulant les groupes Q d'un composé selon une ou plusieurs des revendications 1 à 8, dans lequel ou laquelle la réticulation est en option mise en oeuvre à l'intérieur d'une couche.

10. Procédé pour la fabrication d'une couche réticulée, **caractérisé en ce que** le composé selon une ou plusieurs des revendications 1 à 8 est appliqué à partir d'une solution de manière à obtenir une couche, et cette couche est réticulée.

11. Utilisation d'un composé selon une ou plusieurs des revendications 1 à 8 ou d'un composé ou d'une couche selon la revendication 9 dans un dispositif électronique.

12. Dispositif électronique, en particulier, dispositif électroluminescent organique, circuit intégré organique, transistor à effet de champ organique, transistor à film mince organique, transistor à émission de lumière organique, cellule solaire organique, détecteur optique organique, photorécepteur organique, dispositif à extinction de champ organique, cellule électrochimique à émission de lumière ou diode laser organique, comprenant un ou plusieurs composé(s) selon une ou plusieurs des revendications 1 à 8 ou un composé ou une couche selon la revendication 9.

13. Dispositif électronique selon la revendication 12, lequel est un dispositif électroluminescent organique, comprenant la structure qui suit : une anode/en option une couche qui comprend un polymère conducteur/ une ou plusieurs couche(s) selon la revendication 9/une couche d'émission et une cathode.

14. Dispositif électroluminescent organique selon la revendication 12 ou 13, **caractérisé en ce que** les composés selon une ou plusieurs des revendications 1 à 8 ou les composés selon la revendication 9 sont utilisés dans une couche de transport de trous ou dans une couche d'injection de trous, dans lequel cette couche peut également être dopée, en particulier au moyen d'un composé accepteur d'électrons.

15. Formulation comprenant au moins un composé selon une ou plusieurs des revendications 1 à 8 et un ou plusieurs solvant(s).
